(19) **Europäisches Patentamt**
**European Patent Office**
**Office européen des brevets**

(11) **EP 3 882 363 A1**

(12) **EUROPEAN PATENT APPLICATION**

(43) Date of publication:
**22.09.2021  Bulletin 2021/38**

(51) Int Cl.:
***C12Q 1/6883*** (2018.01)

(21) Application number: **20163627.1**

(22) Date of filing: **17.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BOUWMAN, Wilbert Hendrik**
**5656 AE Eindhoven (NL)**
• **VAN DE STOLPE, Anja**
**5656 AE Eindhoven (NL)**

(74) Representative: **Philips Intellectual Property & Standards**
**High Tech Campus 5**
**5656 AE Eindhoven (NL)**

(54) **PROGNOSTIC PATHWAYS FOR HIGH RISK SEPSIS PATIENTS**

(57)    The present invention relates to means and methods that can determine the functional status of a blood sample of a subject having sepsis, a subject suspected to have sepsis or a subject at risk to develop sepsis. The functional status of the blood sample can be used for diagnosing the subject to have sepsis, or can be used for making a prediction, e.g. whether the subject is likely to develop sepsis, or whether the subject has a high mortality risk as a result of sepsis. The functional status of the blood sample is based on the AR signaling pathway activity, optionally combined with the TGFbeta, MAPK-AP1 and/or JAK-STAT3 signaling pathway activities.

Fig. 1                    GSE26440

**Description**

FIELD OF THE INVENTION

The present invention relates to a method for determining a functional status of a blood sample. The invention further relates to a computer implemented method for determining a functional status of a blood sample and a diagnostic kit useful for implementing the method of the invention.

BACKGROUND OF THE INVENTION

Sepsis has recently been redefined as: infection with organ dysfunction (10). Sepsis is a dysregulated immune response to infection. Sepsis is generally a complication of severe bacterial infection in which bacteria amplify in the bloodstream. This condition is characterized by a systemic inflammatory response, which through a not fully understood mechanisms can lead to septic shock with hypotension that is refractory to fluid resuscitation and hyperlactatemia, progressing organ failure and consequently death. Mortality rates from sepsis range between 25% to 30% for severe sepsis and 40% to 70% for septic shock. The clinical presentation of sepsis is highly variable depending on the etiology, that is, the underlying disease or condition. The most common bacterial sources of infection are the respiratory, genitourinary, and gastrointestinal systems, as well as the skin and soft tissue. Though clinical presentation can be variable, fever with extreme shivering and tachycardia is often the first clinical manifestation of sepsis, with pneumonia and urogenital infection being the most common causes leading to sepsis. Specific treatment consists primarily of giving antibiotics to treat the infection, in addition to standard measures to maintain the blood circulation and prevent hypoxia. However the bacterial cause of the infection is often unknown, so the appropriate antibiotic treatment is an educated guess, until information on the causal bacteria and their sensitivity/resistance pattern to antibiotics becomes available from blood cultures. This information enables adjustment of therapy in case the bacterial species turned out to be resistant to the administered antibiotics. However, blood cultures take long (24 to 48 hours) and turn positive in around 30% of all patients with infections, while, for patients with bacterial sepsis, each hour of delay of administering effective antibiotics increases the relative risk of mortality.

For these reasons many patients progress to septic shock, resulting in a very high mortality for this disease. Besides antibiotics, treatment of septic shock subjects generally consists of intravenous fluid administration and vasopressor therapy. Many additional treatments have been tried to reverse the inflammatory septic shock state, e.g. corticosteroids, however none has proven to be of clinical benefit, although it cannot be excluded that they may benefit a subset of patients who so far cannot yet be identified (10).

One of the reasons is that it is not clear which cellular mechanism(s) is/are responsible for the transition to septic shock and the organ damage. Knowledge on the pathophysiology of sepsis is expected to provide novel drug targets to prevent and treat sepsis in a rational manner.

One of the reasons for failure to develop an effective drug for all is probably the heterogeneity among patients, resulting from the large number of causes and conditions that can cause sepsis and the even larger number of known and unknown factors that influence its progression, while all together determine response or resistance to a drug (e.g. type of bacterial species, genetics, co-existing other diseases, etc.).

The continuum of an inflammatory response to microorganisms is currently classified as infection, sepsis, and septic shock, replacing the older sequence: sepsis, severe sepsis, and septic shock. In addition systemic inflammatory response syndrome (SIRS) was a condition, defined by the presence of two out of four criteria: fever, tachycardia, tachypnea, and leukocytosis or leukopenia, in the absence of (proven) infection; more recently this diagnosis has been abandoned because of lack of sensitivity and specificity .

Clinical criteria used to identify sepsis patients in the hospital who are at high risk of sepsis-related death are (1) changed mental state; (2) systolic blood pressure <100 mmHg, and respiratory rate over 22 per minute. Using these criteria enables improved identification of high risk patients, but cannot identify patients at low risk.

Rationally the best way to reduce death from sepsis is to as early as possible recognize patients at risk for developing sepsis. Such a high risk patient can subsequently be stratified for more intense monitoring (for example at an ICU), identification and elimination of infectious foci (e.g. indwelling catheters), performing more frequent bacterial cultures, administration of preventive antibiotics, and elimination of immunosuppressive factors.

Urinary catheters, open wounds or wounds with drains, intravascular lines etc. are all nearly invariably associated with some extent of bacterial colonization, and therefore may present a risk at sepsis in a susceptible patient. These conditions are present in a major part of the hospitalized patients, and as such they represent a patient population in which clinical outcome may be improved upon early assessment of risk at sepsis and septic shock. Patient genetics and the functional state of the immune system are likely to be relevant factors determining risk.

Susceptibility of a patient with an infection to develop sepsis and progress to septic shock, is in part determined by the immune response that the patient mounts against the infectious pathogen.

The functionality of the immune response (immune active versus immune suppressed, inflammatory immune response versus adaptive T-cell mediated response) is determined by multiple endogenous factors, such as age, genetic variations, comorbidities (e.g. chronic diseases like diabetes), past treatments (like chemotherapy, bone marrow transplantation, corticosteroids), and exogenous factors such as current immunosuppressive treatment.

The consequence of all these factors on the functioning of the immune system in an individual patient cannot yet be assessed. This makes it currently very difficult to predict (1) which patient with an infection will be at risk of developing sepsis, (2) what is the size of that risk, (3) what is the risk at progression to septic shock and ultimately death. Realizing that the state of the immune response is an important determinant of the risk to develop sepsis, may also enable development of novel therapies for patient with an infection or sepsis, based on improving the failing immune function (i.e. immunotherapy).

However due to the large heterogeneity among patients, including gene variants that influence both the immune response and the susceptibility to develop sepsis, the molecular cause for the dysfunctional immune response in sepsis differs between individuals. This implicates that a personalized treatment approach will be necessary, based on characterization of the cellular mechanisms which determine the functional immune status in the individual patient, in order to choose the most effective immune targeting drug with minimal side effects (e.g. a cytokine storm). Personalized treatment based on the genetic profile of a patient has not proven to be effective.

Clearly the immune dysfunction phenotype that is involved in the susceptibility for sepsis is determined not only by the genetic profile, but also to an important extent by the many environmental cues of the patient (e.g. bacterial species, load, comorbidities, medication etc). This implies that the individual functional immune status should be characterized on a phenotypic level, that is activity of cellular mechanisms which control immune functions.

Immune cell functions are orchestrated by highly controlled interactions between signaling pathways, like the TGFbeta, PI3K, MAPK, JAK-STAT, and AR pathways. Recently novel assays were developed to measure activity of signal transduction pathways in a cell or tissue sample, including a blood sample, based on target gene mRNA measurements, that are interpreted by a Bayesian computational models and translated into a pathway activity score (2), (3), (4). Measuring activity of these pathway allows characterization of the functional status of all types of immune cells (5).

The diagnosis sepsis is based on partially subjective clinical criteria, and therefore is not very sensitive, nor specific, mainly due to the large variety in patients (1). When sepsis is suspected based on clinical criteria, it remains important to rapidly confirm the diagnosis, in order to work out the patient care and treatment plan. Confirmation by for example a blood culture is a time consuming process which can take several days, and cannot be waited for.

Faster confirming diagnostic tests are needed, as well as tests to predict which patient with an infection (mainly in the hospital) is at risk of developing sepsis, test to predict the risk to proceed to septic shock, and to predict risk at death. The clinical action that can be taken based on such a test can for example be to stratify subjects for admission to an intensive care unit (ICU) versus remaining at the general ward, to stratify for surgical search for and removal of a source of infection in a high risk patient, to stratify for specific treatment, e.g. targeted immunotherapy.

To summarize, there is a high need for additional tests which can be rapidly performed and can be used to (1) predict sepsis risk in an infected hospital (or at home) patient, to (2) early diagnose sepsis, and to (3) predict risk at progression to septic shock and death; and (4) to predict response to therapy, such as (targeted) therapy or immunotherapy on an individual patient basis (personalized treatment).

SUMMARY OF THE INVENTION

In accordance with a first aspect of the invention, the above problem is solved by a method for determining a functional status of a blood sample, based on RNA extracted from the blood sample, the method comprising the steps of:

- determining or receiving the result of a determination of the expression level of three or more target genes of the AR pathway;
- determining the AR signaling pathway activity, based on the determined expression levels of said three or more target genes of the AR signaling pathway;
- determining the functional status of the blood sample based at least on the determined AR signaling pathway activity, wherein said functional status of said blood sample is being determined as having the determined AR signaling pathway activity,

wherein said blood sample is obtained from a subject with sepsis or obtained from a subject suspected to have sepsis or an subject at risk of developing sepsis.

In a preferred embodiment, the method further comprises,
determining or receiving the result of a determination of the expression level of three or more target genes of the TGFbeta pathway,
determining the TGFbeta signaling pathway activity based on the determined expression levels of said three or more target genes of the TGFbeta signaling pathway,
and wherein said functional status of said blood sample is further based on the determined TGFbeta signaling pathway activity, wherein said functional status is further being determined as having the determined TGFbeta signaling pathway activity.

In a preferred embodiment the method further comprises:

determining or receiving the result of a determination of the expression level of three or more target genes of the MAPK-AP1 signaling pathway, and determining the MAPK-AP1 signaling pathway activity based on said expression levels of the three or more target genes of the MAPK-AP1 signaling pathway, and/or

determining or receiving the result of a determination of the expression level of three or more target genes of the JAK-STAT3 signaling pathway, and determining the JAK-STAT3 signaling pathway activity based on said expression levels of the three or more target genes of the JAK-STAT3 signaling pathway,
and wherein said functional status of said blood sample is further based on the determined MAPK-AP1 signaling pathway activity and/or JAK-STAT3 signaling pathway activity , wherein said functional status is further being determined as having the determined MAPK-AP1 signaling pathway activity, and/or wherein said functional status is further being determined as having the determined JAK-STAT3 signaling pathway activity.

In a further embodiment, the method further comprises providing the functional status of the blood sample obtained from a subject for the purpose of the various uses discloses herein, such as diagnosis, predicting the risk of developing sepsis in a patient with an infection, predicting the risk to progress from sepsis to septic shock or predicting the mortality risk.

The present invention is based on the inventor's innovation that analysis of signal transduction pathway activities can be used to characterize a blood sample, for example a blood sample consisting of at least one immune cell type or a mixed collection of immune cell types, based on determining the activity of at least the AR signaling pathway, and optionally determining the activity or activities of additional pathway(s), such as the TGFbeta pathway, the MAPK-AP1 pathway and the JAK-STAT3 pathway. The inventors demonstrate that based on the AR pathway activity alone, when determined on a blood sample such as a whole blood sample, it can function as a test to establish or confirm the diagnosis of sepsis in a patient with clinical criteria that are suggestive of sepsis. However when a low AR pathway activity is found, sepsis may still be present, but the patient is likely to be a survivor. Further, the level of AR pathway activity can be used to distinguish between low and high, meaning based on blood samples obtained from subjects having sepsis a distinction can be made between samples obtained from a subject having high mortality risk and samples obtained from a subject having low mortality risk, based on the AR pathway activity. Lastly it was found that the correlation between pathway activity and sepsis vs control or low vs high mortality risk was specific for these four pathways, no other signaling pathways were found to correlate with presence or absence of sepsis or severity of sepsis (including mortality risk) in whole blood samples (data not shown).

Important for the invention is that activity of the AR, TGFbeta, MAPK-AP1, and JAK-STAT3 pathways is associated with immunosuppression. The finding that these pathways, especially the AR pathway, are on average abnormally activated in patients with sepsis, is important, because it indicates that a normal activity of this pathway, probably associated with a normal immune function. This appears to be substantiated by the finding of the inventors that the few patients with a normal AR pathway activity, similar to control subjects without infection, are the sepsis-survivors. This appears to suggest that normal immune function is required for survival of sepsis. Overall, it is clear that a good immune response is crucial for prevention of infection and for development of sepsis, and for prevention of sepsis-related mortality. It is again emphasized that it is surprising that this can be determined on the measured signaling pathway activities in whole blood.

The measurement of pathway activity on a blood sample, or on a specific subset of cells from a whole blood sample, provides information on the immune status of a patient with infection. It can be inferred that a patient with an infection, such as a urogenital infection associated with a bladder catheter, will be at higher risk to develop sepsis if the immune response is suppressed. Measuring activity of the AR pathway, and also the TGFbeta, MAPK-AP1, and JAK-STAT3 pathways in a blood sample of a patient with an infection will provide information on the risk for development of sepsis, which is high when activity of these pathways, especially the AR pathway, is increased. This will allow timely allow prediction of sepsis risk in patients with an infection, especially a bacterial infection.

For the first time the inventors demonstrate that determination of pathway activity can be used to diagnose sepsis in a patient and stratify blood samples obtained from a subject to e.g. distinguish between sepsis and septic shock. More surprisingly however is the finding that this analysis can be performed on a blood sample such as a whole blood sample.

The present invention has been accomplished by intensively studying the activities of several signaling pathways such as the AR signaling pathway, the TGFbeta pathway, the MAPK-AP1 pathway and the JAK-STAT3 signaling pathway, in blood samples obtained from healthy control subjects, septic subjects that recovered and septic subjects that passed away as a result from septic shock.

The ability to reliably determine whether a blood sample obtained from a subject or individual is obtained from a subject or individual with sepsis or not is desirable for several reasons. Currently a sepsis diagnosis is initially based on clinical parameters like respiratory rate, heart rate and blood pressure, which can be complemented by simple and not specific clinical chemistry lab measurements, such as lactate, CRP, electrolytes, urea, creatinin. This is not a very accurate diagnosis (not sufficiently sensitive and not specific), therefore when sepsis is suspected in a subject, the diagnosis needs to be confirmed by blood pathogen culture to detect the causative pathogen and profile its antibiotic resistance, which is a time consuming process which may take days to complete. In contrast, determining the functional status of a blood sample by determining one or more pathway activities based on extracted mRNA may be achieved on as little as 2-3 hours.

Once a sepsis diagnosis has been made, either by the methods described here or by other means, it is advantageous to determine whether a subject has a high or a low risk at progression to septic shock, and whether the patient has a high or low mortality risk. Currently, septic subjects, especially septic shock patients, are generally treated in intensive or urgent care units, which is costly. This may not always be necessary, considering the functional status of the blood sample as defined herein may be used to determine the risk at progression to septic shock and the mortality risk of a subject. Therefore once sepsis has been diagnosed, a risk assessment can be performed based on the functional status of the blood sample, for example based on the determined AR pathway activity, to determine whether the subject has a high or a low risk at progression to septic shock or high or low mortality risk. If a subject has low risk at septic shock or low mortality risk, subsequent treatment may not necessarily need to take place in the ICU and thus saving substantial cost. Furthermore, when a subject is determined to be at high risk, treatment in the ICU is beneficial, and additional monitoring or treatment may be warranted to further mitigate the risks, and for example a search for the causative pathogen may be intensified, and the source eradicated.

For the described reasons, the functional status of a blood sample is a useful tool. When used herein, "the functional status of a blood sample" is defined as the combined information of the determined activity or activities of the pathway or pathways from which the activity or activities have been determined. Generally, the activity of a pathway can be determined to be active or not active, or the activity can be determined in reference to control sample. A control sample may be a blood sample obtained from a healthy subject, but it may also refer to samples or data used to calibrate the model used to determine the pathway activity. Therefor a control sample is not necessarily a blood sample, but may also be a different sample with a known functional status of the pathway (i.e. active or not active). By comparing the activity to a reference such as a control sample, the activity may be expressed as a binary value (i.e. the pathway is active or not active), or it may be expressed as a relative value, represented by a number. Therefore, for example when only the AR pathway is determined on a blood sample, the functional status of the blood sample can be either qualified as active AR activity or inactive AR activity in reference to a control sample. Alternatively if the relative value of the pathway activity is represented by a number, and inactive control samples are defined as having a value of 0 and active control samples are defined as having a value of 1, the pathway activity as determined in the blood sample obtained from the subject may for example be 0.81, indicating the pathway activity is closer to active than inactive.

Therefore, the pathway activity based on the determined gene expression levels is preferably expressed as a numeric value. Using a model as described below, the gene expression levels of a pathway can be used to quantify the pathway activity with reference to the calibrated expression levels of the pathway gene and/or with reference to a control sample (e.g. a blood sample obtained from a healthy subject). This quantification can be a simple binary model (e.g. value 0 for pathway inactive, value 1 for pathway active), or can be more complex by quantifying the contribution of each gene from which the expression level has been determined, optionally multiplied by a weight factor. Therefore, "the status of a blood sample" as describe herein is the numeric value of the pathway activity as determined, or if multiple pathway activities are determined, it is the combined numeric values attributed to the determined pathways.

Therefore, preferably the status of the blood sample obtained from a subject comprises one or more activities of a signaling pathway, said signaling pathway activity is preferably: the AR signaling pathway activity, the AR and the TGFbeta signaling pathway activities, The AR and the MAPK-AP1 signaling pathway activities, the AR and the JAK-STAT3 signaling pathway activities, the AR, TGFbeta, and MAPK-AP1 signaling pathway activities, the AR, TGFbeta and JAK-STAT3 signaling pathway activities, the AR, MAPK-AP1 and JAK-STAT3 signaling activities and/or the AR, TGFbeta, MAPK-AP1 and JAK-STAT3 signaling activities, wherein said signaling activities are based on the determined expression levels of three or more target genes of the respective pathways.

In accordance with this, it is an embodiment of the present invention that the determining of the functional status of a blood sample is further based on a respective reference signaling pathway activity or combination of reference activities of signaling pathways. Similarly, the determining of the diagnosis or mortality risk may be further based on a reference activity of the respective signaling pathway. A reference activity reflects activity of the respective signaling pathway found in blood samples obtained from healthy subjects and obtained from septic patients with known clinical outcomes (e.g. septic shock recovered, septic shock died).

For the purpose of the invention determining the expression levels of the target genes based on the extracted RNA may be a part of the method, meaning the method includes the step of determining the expression levels of the target genes on RNA extracted from the blood sample obtained from the patient using methods known to the skilled person or described herein. The method may further include the step of obtaining a blood sample form the patient in order to extract the RNA. Alternatively the expression levels may have been determined separately and the demining step (of the expression levels of the target genes) is not an active step in the method of the invention. In such case the expression levels are provided as an input value, e.g. a relative expression level in reference to one or more control gene expression levels.

By comparing each of the reference pathway activities to each of the respective pathway activities in the subject to be diagnosed, the status of the blood sample comprising each of the respective pathway activities can be determined.

When used herein, "expression level" refers to quantifying the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Sets of cellular signaling pathway target genes whose expression levels are preferably analyzed have been identified, alternatively methods for identifying suitable target genes are described herein. For use to determine pathway activity, for example by a mathematical model, three or more, for example, three, four, five, six, seven, eight, nine, ten, eleven, twelve or more, target genes from each assessed cellular signaling pathway can be analyzed to determine pathway activities.

The blood sample obtained from a subject can be any type of blood sample, that is, the blood may be drawn for example using a cannula and may be whole blood or a defined fraction of blood, such as isolated PBMCs, isolated CD4+ cells, mixed CD8+ and T cells, isolated neutrophils or isolated monocytes. Preferably the sample is whole blood. The presented invention is based on the surprising finding that whole blood, despite the versatility of cells comprised therein, can be used for pathway analysis to determine the functional status of said blood sample, and wherein said functional status of said blood sample can be used for, for example, diagnosis and prognosis of the subject having sepsis or suspected to have sepsis from which the blood sample has been obtained.

The activity of one or more signaling pathways can thus be used as a biomarker that characterizes the functional status of a blood sample, which will be useful for early prediction of the development of sepsis in a patient with an infection, diagnosis of sepsis in subject, predicting the progression to septic shock and mortality risk of a subject with sepsis, and choice of therapy for a subject with sepsis.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The term "subject", as used herein refers to any living being. In some embodiments, the subject is an animal, preferably a mammal. In certain embodiments, the subject is a human being, such as a medical subject. Although the invention is not necessarily limited to a particular group of subjects, it will be apparent that a subject having sepsis or a subject suspected to have sepsis or a subject at risk for developing sepsis profits the most form the invention described herein. It is therefore preferred that the subject from which the blood sample has been obtained is a subject having sepsis, in case sepsis has already been confirmed be alternative means (e.g. a blood culture or by the claimed method), or is a subject suspected to have sepsis or an subject at risk of developing sepsis. A subject suspected to have sepsis may be a subject which meets one or more criteria that define SIRS or sepsis, such presence of fever, tachycardia, tachypnea, and/or leukocytosis or leukopenia. Alternatively a subject suspected to have sepsis may be a subject at risk of having or developing sepsis, e.g. a subject having an infection that may lead to sepsis or a subject having, cancer, diabetes, reduced immunity, a subject having spent prolonged time in the intensive care unit, subject who are born preterm, have a low AGPAR score, etc. The subject may also be a subject at risk of developing sepsis, when used herein a "subject at risk of developing sepsis" refers to a subject which does not currently have sepsis but has one or more increased risk factors which may lead the subject to develop sepsis, for example the presence of urinary catheters, open wounds or wounds with drains, intravascular lines etc.

The blood sample to be used in accordance with the present invention can be an extracted sample, that is, a sample that has been extracted from the subject. Examples of the sample include, but are not limited to whole blood sample, isolated PBMCs, isolated CD4+ cells, mixed CD8+ and T cells, isolated neutrophils or isolated monocytes. Isolated PBMCs, isolated CD4+ cells, mixed CD8+ and T cells, isolated neutrophils or isolated monocytes are generally obtained from whole blood samples be methods known to the skilled person. Further the skilled person is familiar with how to obtain a whole blood sample from the subject using conventional methods to draw blood. The term "sample", as used herein, also encompasses the case where e.g. cells, tissue and/or body fluid have been taken from the subject and, e.g., have been put on a microscope slide or fixative, and where for performing the claimed method a portion of this sample is extracted, e.g., by means of Laser Capture Microdissection (LCM), or by punching, or by scraping off the cells of interest from the slide, or by fluorescence-activated cell sorting techniques. In addition, the term "sample", as used herein, also encompasses the case where e.g. cells, tissue and/or body fluid have been taken from the subject and have been put on a microscope slide, and the claimed method is performed on the slide. Preferably the sample is a body fluid, particularly whole blood, or one or more cell types isolated from a whole blood sample.

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in a intracellular domain.

The term "target gene", as used herein, means a gene whose transcription is directly or indirectly controlled by a respective transcription factor element. The "target gene" may be a "direct target gene" and/or an "indirect target gene" (as described herein).

Pathway analysis enables quantitative measurement of signal transduction pathway activity in blood cells, based on inferring activity of a signal transduction pathway from measurements of mRNA levels of the well-validated direct target genes of the transcription factor associated with the respective signaling pathway (see for example W Verhaegh et al., 2014, supra; W Verhaegh, A van de Stolpe, Oncotarget, 2014, 5(14):5196).

In accordance with this, it is an embodiment of the present invention that the determining of the functional status of a blood sample and/or its subsequent uses such as diagnosing a patient or predicting a mortality risk is further based on a respective combination of reference activities of signaling pathways. Similarly, the determining of the signaling pathway abnormality factor may be further based on a reference activity of the respective signaling pathway. A reference activity reflects activity of the respective signaling pathway found in a blood sample of healthy subjects.

By comparing each of the reference pathway activities to each of the respective pathway activities in the subject to be diagnosed, a functional status of the blood sample comprising each of the respective pathways can be determined. The functional status of the blood sample indicates whether the activity of the respective pathway(s) deviates (abnormally) from the reference activity of the respective pathway(s). The functional status of the blood sample may then be translated into diagnosis or a mortality risk. The functional status of a blood sample may also be computed directly from the combination of pathway activities. The functional status of a blood sample can be considered as multi-pathway score, MPS, and denotes a likelihood that a subject has sepsis, or the risk of mortality as a result of sepsis. Accordingly, the " the functional status of a blood sample ", refers to a dimension, e.g. a level or a value, relating the combination of pathway activities to a likelihood that the subject has sepsis, or the risk of mortality as a result of sepsis.

The term "sepsis" as used herein refers to a condition that arises when the body's response to infection causes injury to its tissues and organs. Sepsis is an inflammatory immune response triggered by an infection. Bacterial infections are the most common cause, but fungal, viral, and protozoan infections can also lead to sepsis. Common locations for the primary infection include the lungs, brain, urinary tract, skin, and abdominal organs.

The functional status of the blood sample is based on a single cellular signaling pathway activity or on a "combination of activities of cellular signaling pathways". This means that the functional status of the blood sample is influenced by the activities of one or more cellular signaling pathways. The activities of the one or more cellular signaling pathways can be inferred and/or combined by a mathematical model as described herein. In a preferred embodiment, the functional status of the blood sample is based on a combination of signaling pathway activities comprising activities of more than 2 cellular signaling pathways. Such combination of signaling pathway activities may include the activities of 3 or 4, or even more than 4 such as 5, 6, 7 or 8, or even more, different signaling pathways.

In general, many different formulas can be devised for determining an functional status of the blood sample that is based on a combination of activities of two or more cellular signaling pathways in a subject, i.e.:

$$MPS = F(Pi) + X,$$

with i = 1...N,

wherein MPS denotes the functional status of the blood sample and/or risk score (the term "MPS" is used herein as an abbreviation for "Multi-Pathway Score" in order to denote that the functional status of the blood sample can be influenced by the activities of two or more cellular signaling pathways), Pi denotes the activity of cellular signaling pathway i, N denotes the total number of cellular signaling pathways used for calculating the functional status of the blood sample, and X is a placeholder for possible further factors and/or parameters that may go into the equation. Such a formula may be more specifically a polynomial of a certain degree in the given variables, or a linear combination of the variables. The weighting coefficients and powers in such a polynomial may be set based on expert knowledge, but typically a training data set with known ground truth, e.g., survival data, is used to obtain estimates for the weighting coefficients and powers of the formula above. The activities may be combined using the formula above and will subsequently generate an MPS. Next, the weighting coefficients and powers of the scoring function may be optimized such that a high MPS correlates with a higher probability that the patient has sepsis and/or has a high mortality risk, and vice versa. Optimizing the scoring function's correlation with known data can be done using a multitude of analysis techniques, e.g., a Cox proportional hazards test (as preferably used herein), a log-rank test, a Kaplan-Meier estimator in conjunction with standard optimization techniques, such as gradient-descent or manual adaptation, and so on.

When used herein, the term "risk score" or "risk factor" general refers to a prediction, risk assessment or diagnosis for a subject based on the functional status of the blood sample. For example the risk score may be the diagnosis of a subject to have sepsis (the risk that the subject is septic), the mortality risk of a septic subject, and/or the risk of developing sepsis for a non-septic subject, the risk of relapsing of a subject which was previously diagnosed with sepsis.

Preferably the determining of the activity or activities of the signaling pathway(s), the combination of multiple pathway activities and applications thereof is performed as described for example in the following documents, each of which is hereby incorporated in its entirety for the purposes of determining activity of the respective signaling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response").

The models have been biologically validated for ER, AR, PI3K-FOXO, HH, Notch, TGF-β, Wnt, NFkB, JAK-STAT1/2, JAK-JAK-STAT3 and MAPK-MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a blood sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a blood sample based on the determined activity level of the signaling pathway associated TF element. As described herein, the activity level can be directly used as an input to determine functional status of the blood sample and/or diagnosis and/or risk score, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., 2014, supra.

To facilitate rapid identification of references, the above-mentioned references have been assigned to each signaling pathway of interest here and exemplarily corresponding target genes suitable for determination of the signaling pathway's activity have been indicated. In this respect, particular reference is also made to the sequence listings for the target genes provided with the above-mentioned references.

AR: KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR and EAF2 (WO 2013/011479, WO 2014/102668); KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR, and EAF2 (WO 2014/174003); TGF-β: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1 and VEGFA (WO 2016/062891, WO 2016/062893); MAPK-AP-1: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53 and VIM (WO 2019/120658); and JAK-JAK-STAT3: AKT1, BCL2, BCL2L1, BIRC5, CCND1, CD274, CDKNIA, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1 (WO 2019/068543).

In an embodiment the signaling pathway measurements are performed using qPCR, multiple qPCR, multiplexed qPCR, ddPCR, RNAseq, RNA expression array or mass spectrometry. For example, gene expression microarray data, e.g. Affymetrix microarray, or RNA sequencing methods, like an Illumina sequencer, can be used.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating the functional status of the blood sample and/or the risk score and the activities of the signaling pathways, or the calibrated mathematical pathway model may be based on one or more linear combination(s) of the activities of the signaling pathways.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as an blood cell present in a sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the blood cell based on the determined activity level of the signaling pathway associated TF element. As described herein, the activity level can be directly used as an input to determine the functional status of a blood sample, a method of diagnosing a subject with sepsis, determining the mortality risk of a subject with sepsis or determining the risk of a subject to develop sepsis, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., 2014, supra.

As a non-limiting example, the following method can be used to generate a model for determining signaling pathway activity: in a plurality of datasets expression RNA levels of different genes are determined in samples where the pathway is assumed to be active and samples where the pathway is assumed to be not active. The expression levels are normalized, for example based on the expression levels of house-keeping genes. Based on the normalized expression levels of the samples where the pathway is assumed to be active or assumed to be inactive, a threshold can be determined, where if the normalized expression level of a gene in a sample is below the threshold the pathway is more likely to inactive and if the expression level is above the threshold the pathway is more likely to be active. Based on this threshold a simple model can be constructed, where a value is assigned to the expression level, as determined in a blood sample of a subject with sepsis or suspected to have sepsis, and the pathway activity is determined as the sum of these values for each gene for which the expression level is determined. Alternatively, the values obtained for each gene for the respective pathway can be compared with values obtained for said gene in a reference blood sample from a healthy subject (i.e. a subject not having sepsis).

According to a preferred embodiment of the present invention
said determining the expression level of three or more target genes of the AR signaling pathway, the TGFbeta signaling pathway, the MAPK-AP1 signaling pathway and/or the JAK-STAT3 signaling pathway comprises:

determining the expression level of three or more target genes, for example 3, 4, 5, 6, 7, 8, 9, 10, 11, 12 or more target genes of the AR signaling pathway selected from the list consisting of: KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR and EAF2, preferably wherein the set of target genes of the AR pathway comprises three or more target genes, for example 3, 4, 5, 6, 7, 8, 9, 10, 11 or 12 target genes selected from the group consisting of ELL2, FKBP5, GUCY1A3, LRIG1, PLAU, PMEPA1, PRKACB, SGK1, NDRG1, CREB3L4, DHCR24 or PTPN1, and/or;
determining the expression level of three or more target genes, for example 3, 4, 5, 6, 7, 8, 9, 10, 11 or more target genes of the TGFbeta signaling pathway comprises determining the expression level of three or more target genes selected from the list consisting of ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, preferably wherein the set of target genes of the TGFbeta pathway comprises three or more target genes, for example 3, 4, 5, 6, 7, 8, 9, 10, 11 or 12 target genes selected from the group consisting of CDC42EP3, GADD45A, ID1, MMP9, SGK1, SMAD5, SMAD7, VEGFA, JUNB, TIMP1, SKIL and CCKN1A, and/or;
determining the expression level of three or more target genes of the MAPK-AP1 signaling pathway comprises determining the expression level of three or more target genes, for example 3, 4, 5, 6, 7, 8, 9, 10, 11, 12 or more target genes selected from the list consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably wherein the set of target genes of the MAPK-AP1 pathway comprises three or more target genes, for example 3, 4, 5, 6, 7, 8, 9, 10, or 11 target genes selected from the group consisting of DNMT1, EGFR, ENPP2, GLRX, MMP9, PLAUR, TIMP1, LOR, EZR, DDIT3 and TP53, and/or;
determining the expression level of three or more target genes, for example 3, 4, 5, 6, 7, 8, 9, 10, 11 or more target genes of the JAK-STAT3 signaling pathway comprises determining the expression level of three or more target genes selected from the list consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1 preferably wherein the set of target genes of the JAK-STAT3 pathway comprises three or more target genes, for example 3, 4, 5, 6, 7, 8, 9, 10, 11, 12 or more target genes selected from the group consisting of BCL2, BIRC5, CD274, FOS, HSPA1A, JUNB, MMP9, STAT1, TIMP1, BCL2L1, HSPA1B, HSP90AB1, HSP90B1, POU2F1 and ICAM1.

Therefore, in a preferred embodiment the method according to the invention further comprises the step of diagnosing the subject from which the blood sample has been obtained, wherein said subject is diagnosed to have sepsis or wherein said subject is diagnosed to not have sepsis based on:

-   a clinical parameter, and
-   the functional status of the blood sample,

the method further comprising comparing the functional status of the blood sample of the subject to at least one functional status of a blood sample obtained from a healthy or non-septic control subject. In a preferred embodiment the subject is diagnosed to have sepsis if the functional status of the blood sample comprises an AR signaling pathway activity which AR signaling pathway activity is determined to be higher than the AR signaling pathway activity determined in the control blood sample obtained from a healthy or non-septic control and the subject from which the blood sample has been obtained has at least one clinical parameter associated with sepsis. It was found by the inventors that the pathway activity determined on a blood sample obtained from a subject can be used to diagnoses said subject to have sepsis or not. As detailed in the experimental data, determining the AR signaling pathway is sufficient to distinguish blood samples obtained from subjects having sepsis and blood samples obtained from healthy individuals. Optionally other pathway activities can be included in the diagnosis, such as the TGFbeta signaling pathway activity, the MAPK-AP1 signaling pathway activity, and/or the JAK-STAT3 signaling pathway activity. Therefore, the functional status of a blood sample as determined herein can be used as a diagnostic tool to quickly diagnose a subject.

Based on the expression level in a blood sample of three or more target genes from the AR pathway, the AR signaling pathway activity, and thus the functional status of the blood sample can be determined. This AR signaling pathway can be expressed as a quantitative value and compared with the AR signaling pathway activity determined on the blood samples obtained from either healthy subjects or known septic subjects. Therefore, the step of diagnosing a subject preferably further includes comparing the functional status of the blood sample of the subject with the functional status of a blood sample obtained from known septic patients. Because the diagnosing step is based on the functional status of the blood sample, this step optionally further uses the determined TGFbeta, MAPK-AP1, and/or JAK-STAT3 signaling pathway activities if they are determined. By including additional pathways the certainty of the diagnosis may be further improved.

The functional status of a blood sample comprises at least the AR signaling pathway activity expressed as a numeric value. Thus by comparing the functional status of a blood sample from a subject, with the functional status of a control blood sample obtained from a healthy subject a diagnosis can be made based on the difference or similarity of the numeric values represented by at least the AR signaling pathway activities. Therefore, said comparing preferably is done using a plurality of functional statuses of reference blood samples to increase accuracy. More preferably said comparing further includes comparing with one or more, preferably a plurality, additional reference functional statuses of blood samples obtained from known septic subjects.

For example when using multiple reference blood samples of healthy individuals, the AR signaling pathway activities can be calculated for each sample, and an average value can be determined. The AR signaling pathway activity of the subject to be diagnosed can be similar to the average reference AR signaling pathway activity (e.g. within 1 or within 2 standard deviations of the calculated average activity), in which case the subject is diagnosed to not have sepsis. Alternatively the AR signaling pathway can be higher compared to the calculated average AR signaling pathway activity (e.g. at least 1 or 2 standard deviation higher than the average value), in which case the subject is diagnosed to have sepsis. Optionally the other pathway activities can be included in this comparison. The above method can be used regardless of the method used to calculate the signaling pathway activity, provided the same method is used for sample from the subject to be diagnosed and the reference samples.

Preferably for the purpose of diagnosing a subject with sepsis, determining the expression level of three or more target genes of the AR signaling pathway, the TGFbeta signaling pathway, the MAPK-AP1 signaling pathway and/or the JAK-STAT3 signaling pathway comprises:

determining the expression level of three or more target genes, for example 3, 4, 5, 6, 7, 8, 9, 10, 11, 12 or more target genes of the AR signaling pathway wherein three or more target genes, e.g. 3, 4 or 5, are selected form the group consisting of: FKBP5, LRIG1, PMEPA1, DHCR24 and LCP1 and/or;
determining the expression level of three or more target genes, for example 3, 4, 5, 6, 7, 8, 9, 10, 11, 12 or more target genes of the TGFbeta signaling pathway wherein three or more target genes, e.g. 3, 4 or 5, are selected form the group consisting of: MMP9, GADD45A, CDC42EP3, TIMP1 and SMAD5, and/or;
determining the expression level of three or more target genes of the MAPK-AP1 signaling pathway comprises determining the expression level of three or more target genes, for example 3, 4, 5, 6, 7, 8, 9, 10, 11, 12 or more target genes of the MAPK-AP1 pathway wherein three or more target genes, e.g. 3, 4 or 5, are selected form the group consisting of: MMP9, TIMP1, DNMT1, FASLG and PLAUR, and/or;
determining the expression level of three or more target genes, for example 3, 4, 5, 6, 7, 8, 9, 10, 11, 12 or more target genes of the JAK-STAT3 signaling pathway wherein three or more target genes, e.g. 3, 4 or 5, are selected form the group consisting of: MMP9, BCL2, TIMP2, HSPA1A and HSPA1AB.

Therefore, the invention further relates to a method for diagnosing a subject, based on RNA extracted from a blood sample obtained from the subject, the method comprising the steps of:

- determining or receiving the result of a determination of the expression level of three or more target genes of the AR pathway;
- determining the AR signaling pathway activity, based on the determined expression levels of said three or more target genes of the AR signaling pathway;
and optionally:
- determining or receiving the result of a determination of the expression level of three or more target genes of the TGFbeta pathway;
- determining the TGFbeta signaling pathway activity, based on the determined expression levels of said three or more target genes of the TGFbeta signaling pathway;
- determining or receiving the result of a determination of the expression level of three or more target genes of the MAPK-AP1 pathway;

- determining the MAPK-AP1 signaling pathway activity, based on the determined expression levels of said three or more target genes of the MAPK-AP1 signaling pathway;
- determining or receiving the result of a determination of the expression level of three or more target genes of the JAK-STAT3 pathway;
- determining the JAK-STAT3 signaling pathway activity, based on the determined expression levels of said three or more target genes of the JAK-STAT3 signaling pathway;

wherein said method further comprises the step of diagnosing the subject from which the blood sample has been obtained, wherein said subject is diagnosed to have sepsis or wherein said subject is diagnosed to not have sepsis based on:

- a clinical parameter, and
- the activity of the determined signaling pathways in the blood sample,

the method further comprising comparing the activity of the determined signaling pathways in the blood sample of the subject to at the activity of the determined signaling pathways in at least one blood sample obtained from a healthy or non-septic control subj ect,
wherein said blood sample is obtained from a subject with sepsis or obtained from a subject suspected to have sepsis or an subject at risk of developing sepsis. In a preferred embodiment the subject is diagnosed to have sepsis if AR signaling pathway activity which AR signaling pathway activity is determined to be higher than the AR signaling pathway activity determined in the control blood sample obtained from a healthy or non-septic control and the subject from which the blood sample has been obtained has at least one clinical parameter associated with sepsis.

Therefore, in a preferred embodiment of the invention the subject is diagnosed to have sepsis if the functional status of the blood sample comprises an AR signaling pathway activity which AR signaling pathway activity is determined to be higher than the AR signaling pathway activity determined in the reference blood sample obtained from a healthy subject. Alternatively the comparison can further include reference blood samples obtained from known septic subjects. In such case the functional status of the blood sample of the subject can be compared with both the functional status of blood samples of healthy individuals and the functional status of blood samples of known septic subjects. In such case the numeric values assigned to the AR signaling pathway activity (and other pathway activities, if determined) can be compared. For example when the subject has an AR signaling pathway which is close to the average value for known septic subjects, e.g. within 1 or 2 standard deviations, the subject is diagnosed with sepsis, or when the value is close to the average value for healthy subjects, e.g. within 1 or 2 standard deviations, the subject is diagnosed to be non-septic. Alternatively, a statistical method can be used to determine whether the subject is more likely to be in the non-septic or septic group (meaning, the numeric value assigned to the AR signaling pathway is closer to the normal (healthy) average or closer to the septic average value). Other signaling pathway activities can optionally be included in this calculation. When using multiple signaling pathway activities for the diagnosis step, e.g. both AR signaling pathway activity and TGFbeta signaling pathway activity, a clustering method may be used to cluster the healthy control subjects, the known septic subject and the subject to be diagnosed, based on the pathway activities, in order to establish whether the subject to be diagnosed falls within the healthy or the septic group. When used herein, reference blood sample form known septic subjects are blood sample obtained from subjects in which later the diagnosis sepsis has been confirmed, for example by blood culture.

In a preferred embodiment of the method according to the invention, said functional status of the blood sample is used in predicting the mortality risk for the subject from which the blood sample has been obtained,
wherein said prediction is based on a comparison of the functional status of the blood sample of the subject with a plurality of reference functional statuses of the blood samples obtained from reference subjects, wherein said plurality of reference functional statuses of the blood samples obtained from reference subjects comprises at least one functional status of blood sample obtained from subject with sepsis which is a non-survivor and at least one functional status of blood samples obtained from subject with sepsis which is a survivor, and optionally further comprises at least one functional status of blood samples obtained from a healthy or non-septic control subject,
wherein the subject from which the blood sample is obtained is confirmed to have sepsis, and
wherein a low mortality risk is predicted when the functional status of the blood sample obtained from the subject with sepsis is similar to the at least one functional status of the blood sample obtained from reference subject with sepsis which is a survivor or when the functional status of the blood sample obtained from the subject with sepsis is similar to the at least one functional status of the blood sample obtained from the at least one healthy or non-septic control subject, and
wherein a high mortality risk is predicted when the functional status of the blood sample obtained from the subject with sepsis is similar to the at least one functional status of the blood sample obtained from the reference subject with sepsis which is a non-survivor.

In a preferred embodiment said comparing of the functional status of the blood sample obtained from the subject with a plurality of functional statuses of the blood samples obtained from control subjects is performed using clustering of the determined pathway activities, preferably by hierarchical clustering.

It was found by the inventors that a prediction regarding the mortality risk can be made based on the functional status of a blood sample obtained from a subject. By comparing the functional status of a blood sample from a subject with reference samples of septic subject which have died and which have survived, a likelihood can be calculated that the subject will die from sepsis. This prediction can be done by using the numeric values representing the different determined signaling pathway activities, e.g. the AR singling activity, or the combination of AR and TGFbeta signaling activity) and comparing these values with the values obtained for the reference groups. This comparison can be done using statistical methods or for example by using clustering. When using clustering the subject is predicted to have a high mortality risk when the subject is clustered together with the reference subjects which have died, or is predicted to have low mortality risk when clustered together with reference subjects which have survived.

Preferably, the prediction is made in the method according to the invention, wherein the method is performed on the blood sample obtained from a patient having sepsis. Preferably the prediction is made for a subject for which it has already been established that the subject has sepsis, for example by the methods described herein.

Preferably, the prediction is made in the method according to the invention, wherein said comparing of the functional status of the blood sample obtained from the subject with a plurality of functional statuses of the blood samples obtained from control subjects is performed using clustering of the determined pathway activities, preferably by hierarchical clustering. It was found by the inventors that by clustering based on the determined signaling activities samples obtained from septic patients can be identified that have dies as a result form sepsis. Therefore by comparing the determined signaling activities determined in a blood sample obtained from a subject with known reference patients the mortality risk can be predicted as high when the respective signaling activities cluster with patients that have died or the risk can be predicted as low when the respective signaling activities cluster with patients that survived.

Preferably for the purpose of predicting survival probability of a subject with sepsis, determining the expression level of three or more target genes of the AR signaling pathway, the TGFbeta signaling pathway, the MAPK-AP1 signaling pathway and/or the JAK-STAT3 signaling pathway comprises:

determining the expression level of three or more target genes, for example 3, 4, 5, 6, 7, 8, 9, 10, 11, 12 or more target genes of the AR signaling pathway wherein three or more target genes, e.g. 3, 4 or 5, are selected form the group consisting of: ELL2, FKBP5, EAF2, NDRG1 and DHCR24 and/or;

determining the expression level of three or more target genes, for example 3, 4, 5, 6, 7, 8, 9, 10, 11, 12 or more target genes of the TGFbeta signaling pathway wherein three or more target genes, e.g. 3, 4 or 5, are selected form the group consisting of: ID1, SKIL, GADD45A, HMGA2 and SMAD4, and/or;

determining the expression level of three or more target genes of the MAPK-AP1 signaling pathway comprises determining the expression level of three or more target genes, for example 3, 4, 5, 6, 7, 8, 9, 10, 11, 12 or more target genes of the MAPK-AP1 pathway wherein three or more target genes, e.g. 3, 4 or 5, are selected form the group consisting of: BCL2L11, EZR, ENPP2, MMP3 and PLAUR, and/or;

determining the expression level of three or more target genes, for example 3, 4, 5, 6, 7, 8, 9, 10, 11, 12 or more target genes of the JAK-STAT3 signaling pathway wherein three or more target genes, e.g. 3, 4 or 5, are selected form the group consisting of: BIRC5, HSP90B1, MMP3, IL10, HIF1A.

Therefore in an embodiment the invention relates to a method for determining the mortality risk for a subject, based on RNA extracted from a blood sample form the subject, the method comprising the steps of:

- determining or receiving the result of a determination of the expression level of three or more target genes of the AR pathway;
- determining the AR signaling pathway activity, based on the determined expression levels of said three or more target genes of the AR signaling pathway;
  and optionally:
- determining or receiving the result of a determination of the expression level of three or more target genes of the TGFbeta pathway;
- determining the TGFbeta signaling pathway activity, based on the determined expression levels of said three or more target genes of the TGFbeta signaling pathway;
- determining or receiving the result of a determination of the expression level of three or more target genes of the MAPK-AP1 pathway;
- determining the MAPK-AP1 signaling pathway activity, based on the determined expression levels of said three or more target genes of the MAPK-AP1 signaling pathway;
- determining or receiving the result of a determination of the expression level of three or more target genes of the JAK-STAT3 pathway;

- determining the JAK-STAT3 signaling pathway activity, based on the determined expression levels of said three or more target genes of the JAK-STAT3 signaling pathway;

wherein the signaling pathway activities in the blood sample is used in predicting the mortality risk for the subject from which the blood sample has been obtained,
wherein said prediction is based on a comparison of the signaling pathway activities in the blood sample of the subject with the signaling pathway activities in a plurality of blood samples obtained from reference subjects, wherein said signaling pathway activities in a plurality of blood samples obtained from reference subjects comprise at least one blood sample obtained from subject with sepsis which is a non-survivor and at least one blood samples obtained from subject with sepsis which is a survivor, and optionally further comprise at least one blood samples obtained from a healthy or non-septic control subject,
wherein the subject from which the blood sample is obtained is confirmed to have sepsis, and
wherein a low mortality risk is predicted when the signaling pathway activity in the blood sample obtained from the subject with sepsis is similar to the signaling pathway activity in at least one blood sample obtained from reference subject with sepsis which is a survivor or when the signaling pathway activity in the blood sample obtained from the subject with sepsis is similar to the signaling pathway activity in at least one of the blood samples obtained from the healthy or non-septic control subject, and
wherein a high mortality risk is predicted when the signaling pathway activity in the blood sample obtained from the subject with sepsis is similar to the signaling pathway activity in at least one blood sample obtained from the reference subject with sepsis which is a non-survivor.

In a preferred embodiment said comparing of the signaling pathway activities in the blood sample obtained from the subject with a the signaling pathway activities in a plurality of blood samples obtained from control subjects is performed using clustering of the determined pathway activities, preferably by hierarchical clustering.

In a further preferred embodiment the subject from which the blood sample has been obtained does not have sepsis, and wherein the functional status of the blood sample is used to determine the risk that the subject will develop sepsis,
the method further comprising comparing the functional status of the blood sample of the subject from which the blood sample has been obtained to at least one functional status of a blood sample obtained from a healthy or non-septic control subject,
preferably wherein the subject from which the blood sample has been obtained is predicted to be at risk to develop sepsis if the functional status of the blood sample comprises an AR signaling pathway activity which AR signaling pathway activity is determined to be higher than the AR signaling pathway activity determined in the control blood sample obtained from a healthy or non-septic control subject. In a preferred embodiment the predicting the risk to develop sepsis for a subject that does not have sepsis is further based on the TGFbeta signaling pathway activity wherein the subject is at risk to develop sepsis when the TGFbeta signaling pathway activity is determined to be higher than the TGFbeta signaling pathway activity determined in the control blood sample obtained from a healthy or non-septic control subject. In a more preferred embodiment the subject from which the blood sample has been obtained does not have sepsis, is determined to be at risk to develop sepsis when both the AR and the TGFbeta signaling pathway activities are determined to be higher than the AR and TGFbeta signaling pathway activities determined in the control blood sample obtained from a healthy or non-septic control subject. Preferably the subject that does not have sepsis is a subject with a bacterial infection.

It was found by the inventors that in patients that do not have sepsis but are at risk to develop sepsis, e.g. patients with a bacterial infection, patients with a high risk to develop sepsis can be identified as high risk correlates with increased AR and optionally TGFbeta signaling pathway activity.

Therefore, the invention further relates to a method for determining the risk for a non-septic subject to develop sepsis, based on RNA extracted from a blood sample form the subject, the method comprising the steps of:

- determining or receiving the result of a determination of the expression level of three or more target genes of the AR pathway;
- determining the AR signaling pathway activity, based on the determined expression levels of said three or more target genes of the AR signaling pathway;
and optionally:
- determining or receiving the result of a determination of the expression level of three or more target genes of the TGFbeta pathway;
- determining the TGFbeta signaling pathway activity, based on the determined expression levels of said three or more target genes of the TGFbeta signaling pathway; and/or
- determining or receiving the result of a determination of the expression level of three or more target genes of the MAPK-AP1 pathway;
- determining the MAPK-AP1 signaling pathway activity, based on the determined expression levels of said three or more target genes of the MAPK-AP1 signaling pathway; and/or

- determining or receiving the result of a determination of the expression level of three or more target genes of the JAK-STAT3 pathway;
- determining the JAK-STAT3 signaling pathway activity, based on the determined expression levels of said three or more target genes of the JAK-STAT3 signaling pathway;

wherein the signaling pathway activities in the blood sample is used to determine the risk that the subject will develop sepsis,

wherein the subject from which the blood sample has been obtained does not have sepsis,

the method further comprising comparing the signaling pathway activity in the blood sample of the subject from which the blood sample has been obtained to the signaling pathway activity in at least one blood sample obtained from a healthy or non-septic control subj ect.

Preferably the subject that does not have sepsis is a subject with a bacterial infection.

In a preferred embodiment the subject from which the blood sample has been obtained is predicted to be at risk to develop sepsis if the AR signaling pathway activity is determined to be higher than the AR signaling pathway activity determined in the control blood sample obtained from a healthy or non-septic control subject.

In a further preferred embodiment of the invention the subject from which the blood sample has been obtained has recovered from sepsis, and wherein the functional status of the blood sample is used to monitor the risk that the subject will develop a recurrence of sepsis,

the method further comprising comparing the functional status of the blood sample of the subject from which the blood sample has been obtained to at least one functional status of a blood sample obtained from a healthy or non-septic control subject,

preferably wherein the subject from which the blood sample has been obtained is predicted to be at risk to develop a recurrence of sepsis if the functional status of the blood sample comprises an AR signaling pathway activity which AR signaling pathway activity is determined to be higher than the AR signaling pathway activity determined in the control blood sample obtained from a healthy or non-septic control subject. In a preferred embodiment the predicting the risk to develop a recurrence of sepsis for a subject that has recovered from sepsis is further based on the TGFbeta signaling pathway activity wherein the subject is at risk to develop a recurrence of sepsis when the TGFbeta signaling pathway activity is determined to be higher than the TGFbeta signaling pathway activity determined in the control blood sample obtained from a healthy or non-septic control subject. In a more preferred embodiment the subject that has recovered from sepsis is determined to be at risk to develop a recurrence of sepsis when both the AR and the TGFbeta signaling pathway activities are determined to be higher than the AR and TGFbeta signaling pathway activities determined in the control blood sample obtained from a healthy or non-septic control subject.

It was found that after recovery of sepsis patients remain susceptible to develop a recurrence of sepsis for a prolonged time. The inventors demonstrate that higher risk of recurrence correlates with increased AR and TGFbeta signaling pathway activities, and that based on AR signaling pathway activity alone or when combined with TGFbeta signaling pathway activity subject with a risk of developing a recurrence of sepsis can be identified.

Therefore, in a preferred embodiment the invention relates to a method for determining the risk for recurrence for a subject recovered from sepsis, based on RNA extracted from a blood sample form the subject, the method comprising the steps of:

- determining or receiving the result of a determination of the expression level of three or more target genes of the AR pathway;
- determining the AR signaling pathway activity, based on the determined expression levels of said three or more target genes of the AR signaling pathway;
  and optionally:
- determining or receiving the result of a determination of the expression level of three or more target genes of the TGFbeta pathway;
- determining the TGFbeta signaling pathway activity, based on the determined expression levels of said three or more target genes of the TGFbeta signaling pathway; and/or
- determining or receiving the result of a determination of the expression level of three or more target genes of the MAPK-AP1 pathway;
- determining the MAPK-AP1 signaling pathway activity, based on the determined expression levels of said three or more target genes of the MAPK-AP1 signaling pathway; and/or
- determining or receiving the result of a determination of the expression level of three or more target genes of the JAK-STAT3 pathway;
- determining the JAK-STAT3 signaling pathway activity, based on the determined expression levels of said three or more target genes of the JAK-STAT3 signaling pathway;

wherein the subject from which the blood sample has been obtained has recovered from sepsis, and wherein the signaling pathway activity in the blood sample is used to monitor the risk that the subject will develop a recurrence of sepsis, the method further comprising comparing the signaling pathway activity in the blood sample of the subject from which the blood sample has been obtained to the signaling pathway activity in at least one blood sample obtained from a healthy or non-septic control subj ect.

In a preferred embodiment the subject from which the blood sample has been obtained is predicted to be at risk to develop a recurrence of sepsis if the AR signaling pathway activity is determined to be higher than the AR signaling pathway activity determined in the control blood sample obtained from a healthy or non-septic control subject.

According to a preferred embodiment of the present invention the blood sample is a whole blood sample, isolated peripheral blood mononuclear cells (PBMCs), isolated CD4+ cells, isolated CD8+ cells, Regulatory T-cells, mixed CD8+ and T cells, myeloid derived suppressor cells (MDSC), dendritic cells, isolated neutrophils, isolated lymphocytes or isolated monocytes.

In a preferred embodiment of the present invention said signaling pathway activity or signaling pathway activities is determined based on evaluating a calibrated mathematical model relating the to the three or more expression levels determined for the pathway or pathways based on the RNA extracted from a blood sample to the activity or activities of the signaling pathway or signaling pathways.

According to a preferred embodiment of the invention, the functional status of a blood sample is determined based on evaluating a calibrated mathematical model relating the activities of the signaling pathways in the blood sample to a numeric value. This model may be programmed to interpret the combination of pathway activities so as to determine the functional status of the blood sample of the subject to be diagnosed, and optionally further use this functional status to provide a diagnosis or mortality risk. In particular, the determination of the functional status of a blood sample comprises (i) receiving activity of the respective signaling pathways in the blood sample of the subject to be diagnosed, (ii) determining the functional status of the blood sample of said subject, the determining being based on evaluating a calibrated mathematical model relating the activity of the respective signaling pathways to the functional status of the blood sample.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating the functional status of the blood sample and the activities of the signaling pathways, or the calibrated mathematical pathway model may be based on one or more linear combination(s) of the activities of the signaling pathways.

In accordance with the mathematical model, the activities of the signaling pathways are interpreted to provide the functional status of the blood sample, which may further be translated into the diagnosis, or are interpreted to provide directly the diagnosis. The functional status of the blood sample predicts or provides a probability that a subject has sepsis, and/or the probability that a subject with sepsis will die as a consequence of septic shock.

Accordingly, the determining of the diagnosis or determining the mortality risk may comprise determining functional status of the blood sample based on the combination of the activities of the cellular signaling pathways in the blood sample and translating the functional status into the diagnosis or mortality risk. According to a preferred embodiment of the present invention, the activity of the respective signal pathway is determined or determinable by pathway analysis as described herein.

Accordingly in a preferred embodiment of the invention, the method comprises a step of providing a blood sample obtained from a subject, and extracting RNA from said blood sample.

In a preferred embodiment of the invention the subject is a pediatric subject.

In a second aspect, the present invention relates to a computer-implemented method for implementing the method of the first aspect of the invention and various embodiment thereof.

In accordance with a third aspect, the present invention relates to an apparatus for determining the functional status of a blood sample, and/or diagnosing a subject with sepsis, and/or predicting the mortality risk for the subject, the apparatus comprising a digital processor configured to perform the method of the first aspect of the present invention and the various embodiments thereof. In a preferred embodiment the invention relates to an apparatus for determining the functional status of a blood sample, the apparatus comprising a digital processor configured to perform the method according to any one of the preceding claims, comprising an input adapted to receive data indicative of a target gene expression profile for the three or more target genes of the AR signaling pathway, optionally data indicative of a target gene expression profile for the three or more target genes of the TGFbeta signaling pathway and/or the MAPK-AP1 signaling pathway and/or the JAK-STAT3 signaling pathway

In accordance with a fourth aspect, the present invention relates to a non-transitory storage medium for determining the functional status of a blood sample, and/or diagnosing a subject with sepsis, and/or predicting the mortality risk for the subject, the non-transitory storage medium storing instructions that are executable by a digital processing device to perform the method of the first aspect of the present invention and the various embodiments thereof. In a preferred embodiment the invention relates to a computer program product comprising instructions which, when the program is executed by a computer, cause the computer to carry out a method comprising:

- receiving data indicative of a target gene expression profile for three or more target genes of the AR signaling pathway, optionally further receiving data indicative of the target gene expression levels of three or more target genes of the TGFbeta signaling pathway and/or the MAPK-AP1 signaling pathway and/or the JAK-STAT3 signaling pathway,
- determining the AR signaling pathway activity, and optionally TGFbeta signaling pathway activity and/or the MAPK-AP1 signaling pathway activity and/or the JAK-STAT3 signaling pathway activity based on the determined expression levels of said three or more target genes of the AR signaling pathway and optionally the TGFbeta signaling pathway and/or the MAPK-AP1 signaling pathway and/or the JAK-STAT3 signaling pathway,
- determining the functional status of the blood sample based on the determined AR signaling pathway activity and optionally TGFbeta signaling pathway activity and/or the MAPK-AP1 signaling pathway activity and/or the JAK-STAT3 signaling pathway activity, wherein said functional status of said blood sample is being determined as having the determined AR signaling pathway activity and optionally the TGFbeta signaling pathway activity and/or the MAPK-AP1 signaling pathway activity and/or the JAK-STAT3 signaling pathway activity, and
- optionally providing a diagnosis or prediction based on the functional status of the blood sample.

The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with a fifth aspect, the present invention relates to a computer program for determining the functional status of a blood sample, and/or diagnosing a subject with sepsis, and/or predicting the mortality risk for the subject, the computer program comprising program code means for causing a digital processing device to perform a method according to the first aspect of the present invention and the various embodiments thereof, when the computer program is run on the digital processing device. The computer program may be stored/distributed on a suitable medium, such as an optical storage medium or a solid-state medium, supplied together with or as part of other hardware, but may also be distributed in other forms, such as via the Internet or other wired or wireless telecommunication systems.

In a sixth aspect the invention relates to a kit of parts, comprising primers for inferring activity of one or more cellular signaling pathway(s) by determining the expression levels of one or more set(s) of target genes of the respective cellular signaling pathway(s), wherein the cellular signaling pathway(s) comprise(s) a AR pathway, and optionally further comprises one or more of an TGFbeta pathway, an MAPK-AP1 pathway and a JAK-STAT3 pathway, wherein the set of target genes of the AR pathway comprises three or more target genes, for example 3, 4, 5, 6, 7, 8, 9, 10, 11, 12 or more target genes selected from the group comprising: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, preferably wherein the set of target genes of the AR pathway comprises three or more target genes, for example 3, 4, 5, 6, 7, 8, 9, 10, 11 or 12 target genes selected from the group consisting of ELL2, FKBP5, GUCY1A3, LRIG1, PLAU, PMEPA1, PRKACB, SGK1, NDRG1, CREB3L4, DHCR24 or PTPN1, and wherein the set of target genes of the TGFbeta pathway comprises three or more target genes, for example 3, 4, 5, 6, 7, 8, 9, 10, 11, 12 or more target genes selected from the group comprising: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, preferably wherein the set of target genes of the TGFbeta pathway comprises three or more target genes, for example 3, 4, 5, 6, 7, 8, 9, 10, 11 or 12 target genes selected from the group consisting of CDC42EP3, GADD45A, ID1, MMP9, SGK1, SMAD5, SMAD7, VEGFA, JUNB, TIMP1, SKIL and CCKN1A, and wherein the set of target genes of the MAPK-AP1 pathway comprises three or more target genes, for example 3, 4, 5, 6, 7, 8, 9, 10, 11, 12 or more target genes selected from the group comprising: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably wherein the set of target genes of the MAPK-AP1 pathway comprises three or more target genes, for example 3, 4, 5, 6, 7, 8, 9, 10, or 11 target genes selected from the group consisting of DNMT1, EGFR, ENPP2, GLRX, MMP9, PLAUR, TIMP1, LOR, EZR, DDIT3 and TP53, and wherein the set of target genes of the JAK-STAT3 pathway comprises three or more target genes, for example 3, 4, 5, 6, 7, 8, 9, 10, 11, 12 or more target genes selected from the group comprising: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1 preferably wherein the set of target genes of the JAK-STAT3 pathway comprises three or more target genes, for example 3, 4, 5, 6, 7, 8, 9, 10, 11, 12 or more target genes selected from the group consisting of BCL2, BIRC5, CD274, FOS, HSPA1A, JUNB, MMP9, STAT1, TIMP1, BCL2L1, HSPA1B, HSP90AB1, HSP90B1, POU2F1 and ICAM1.

In a preferred embodiment of the invention the kit of parts further comprises the apparatus according to the third aspect of the invention, and/or the non-transitory storage product according to the fourth aspect of the invention, and/or the computer program according to the fifth aspect of the invention.

In a seventh aspect the invention relates to a method for in vivo or ex vitro diagnosing or prognosticating whether a subject has sepsis, has septic shock or has a high mortality risk as a result of sepsis using a kit, the kit comprising primers for inferring activity of one or more cellular signaling pathway(s) by determining the expression levels of one or more set(s) of target genes of the respective cellular signaling pathway(s), wherein the cellular signaling pathway(s) comprise(s) a AR pathway, and optionally further comprises one or more of an TGFbeta pathway, an MAPK-AP1 pathway and a JAK-STAT3 pathway,

wherein the set of target genes of the AR pathway comprises three or more target genes selected from the group comprising: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, and

wherein the set of target genes of the TGFbeta pathway comprises three or more target genes selected from the group comprising: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, and

wherein the set of target genes of the MAPK-AP1 pathway comprises three or more target genes selected from the group comprising: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, and

wherein the set of target genes of the JAK-STAT3 pathway comprises three or more target genes selected from the group comprising: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the methods of the first aspect, the computer implemented invention of the second aspect, the apparatus of the third aspect, the non-transitory storage medium of fourth aspect, the computer program of the fifth aspect, the kits of the sixth aspect have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the mortality risk performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

General: In all the figures where signal transduction pathway analysis scores are depicted, these are given as log2odds scores for pathway activity, derived from the probability scores for pathway activity provided by the Bayesian pathway model analysis. Log2odds scores indicate the level of activity of a signaling pathway on a linear scale.

Analyzed public datasets are indicated with their GSE number (in principle at the bottom of each figure), and individual samples with their GSM number (in principle most right column for clustering diagrams).

All validation samples for a signaling pathway model or an immune response/system model are independent samples and have not been used for calibration of the respective model to be validated.

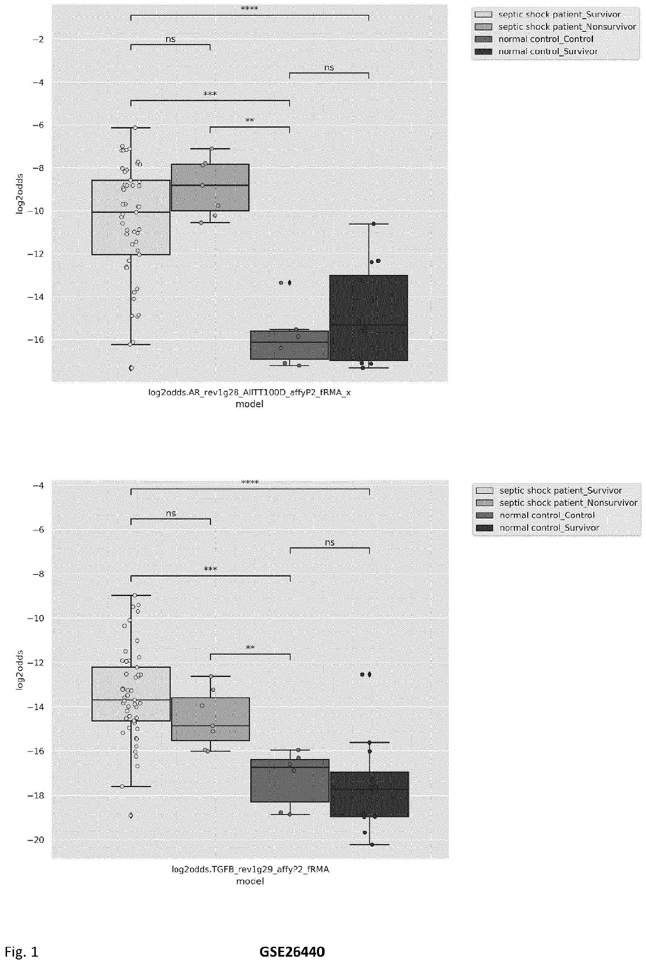

Fig. 1 shows AR signaling pathway activity (top) and TGFbeta signaling pathway activity (bottom) for septic shock patients and healthy control subjects from dataset GSE26440. The data are obtained from whole blood samples from septic shock patients (survivors), septic shock patients (non-survivors), control subject (healthy subjects) and control subjects (non-septic survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

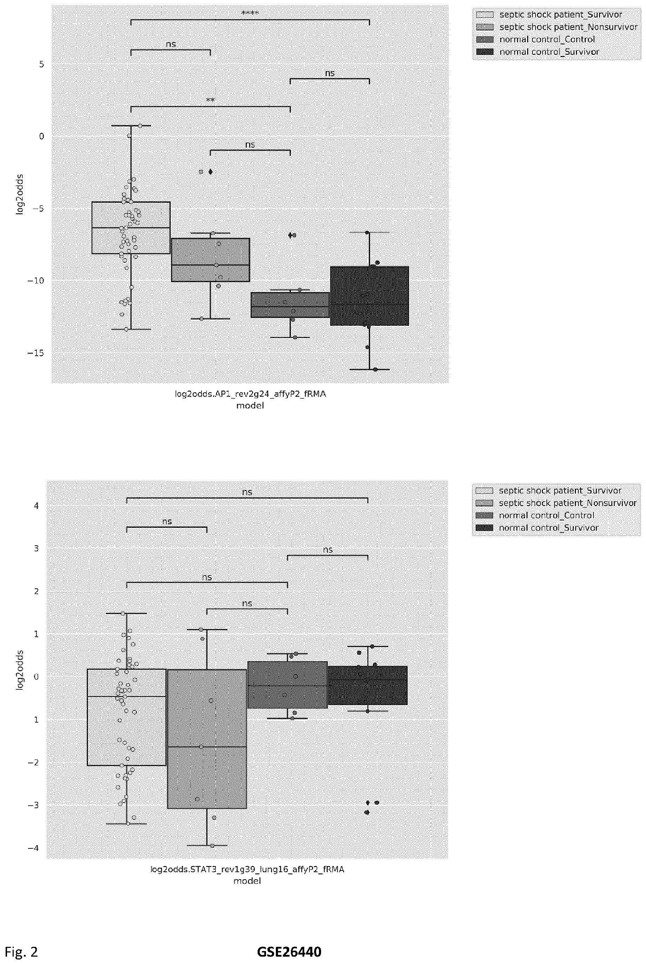

Fig. 2 shows MPK-AP1 signaling pathway activity (top) and JAK-STAT3 signaling pathway activity (bottom) for septic shock patients and healthy control subjects from dataset GSE26440. The data are obtained from whole blood samples from septic shock patients (survivors), septic shock patients (non-survivors), control subject (healthy subjects) and control subjects (non-septic survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

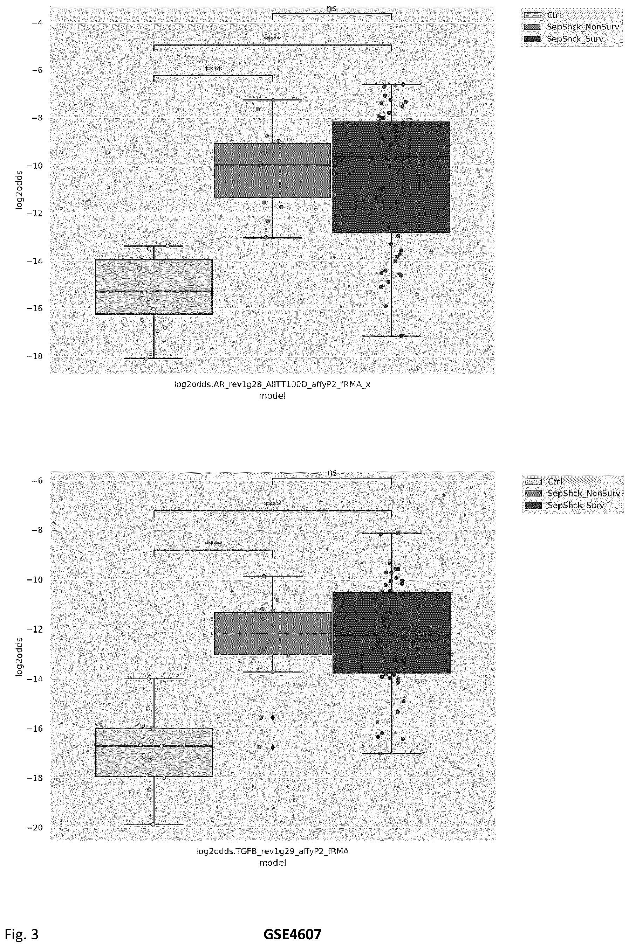

Fig. 3 shows AR signaling pathway activity (top) and TGFbeta signaling pathway activity (bottom) for septic shock patients and healthy control subjects from dataset GSE4607. The data are obtained from whole blood samples from control subjects, septic shock patients (Non-survivors) and septic shock patients (Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

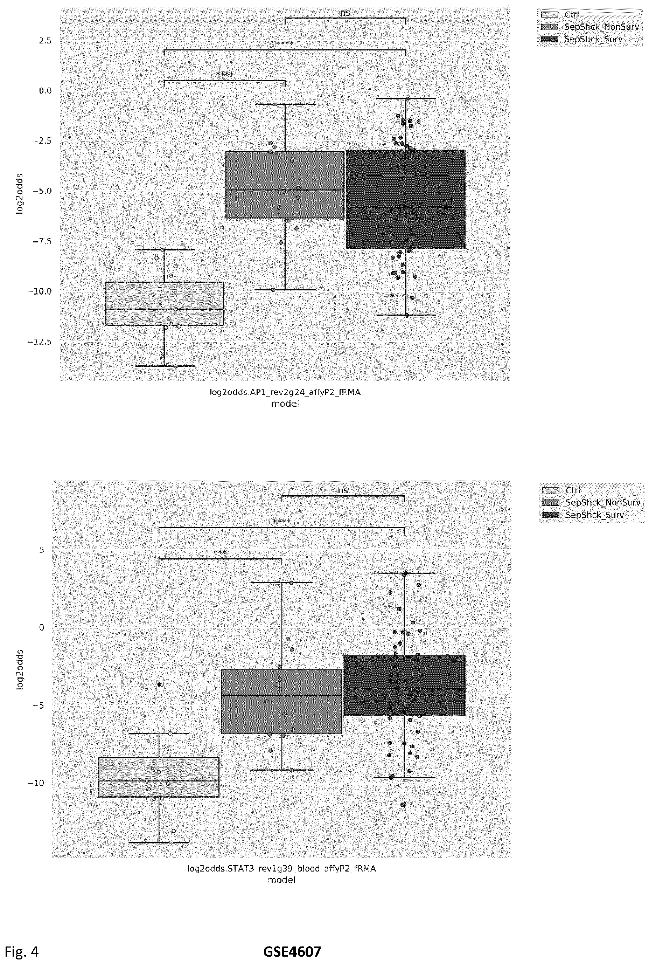

Fig. 4 shows MPK-AP1 signaling pathway activity (top) and JAK-STAT3 signaling pathway activity (bottom) for septic shock patients and healthy control subjects from dataset GSE4607. The data are obtained from whole blood samples from control subjects, septic shock patients (Non-survivors) and septic shock patients (Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

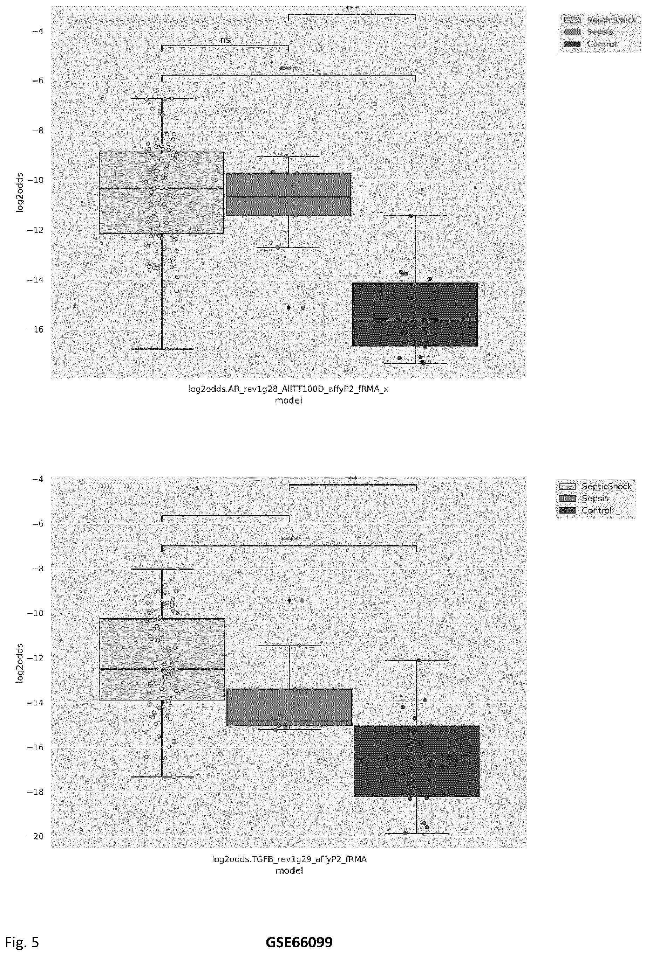

Fig. 5 shows AR signaling pathway activity (top) and TGFbeta signaling pathway activity (bottom) for septic shock patients and healthy control subjects from dataset GSE66099. The data are obtained from whole blood samples from control subjects, septic shock patients (Non-survivors) and septic shock patients (Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

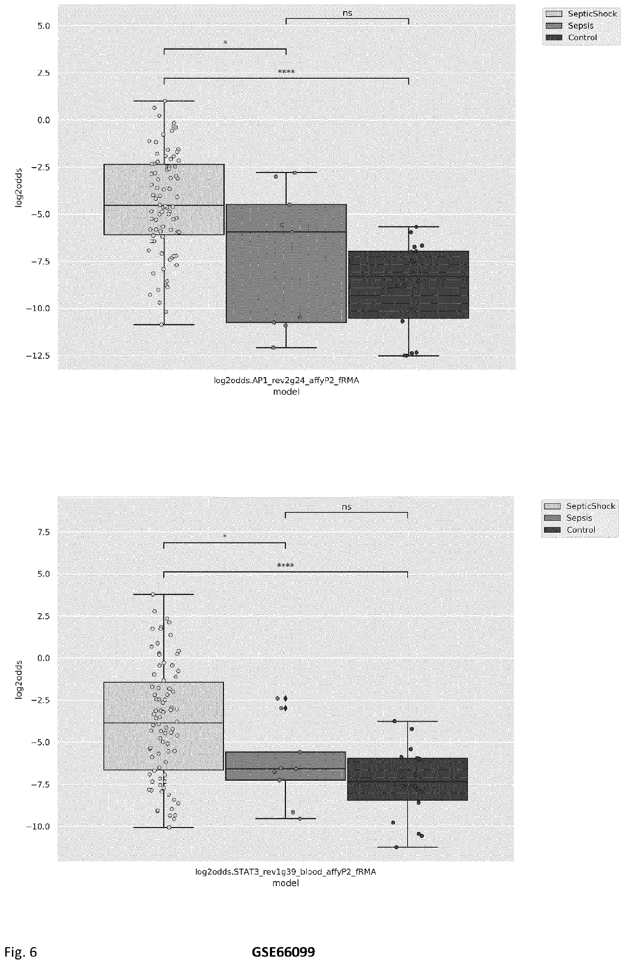

Fig. 6 shows MPK-AP1 signaling pathway activity (top) and JAK-STAT3 signaling pathway activity (bottom) for septic shock patients and healthy control subjects from dataset GSE66099. The data are obtained from whole blood samples from control subjects, septic shock patients (Non-survivors) and septic shock patients (Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

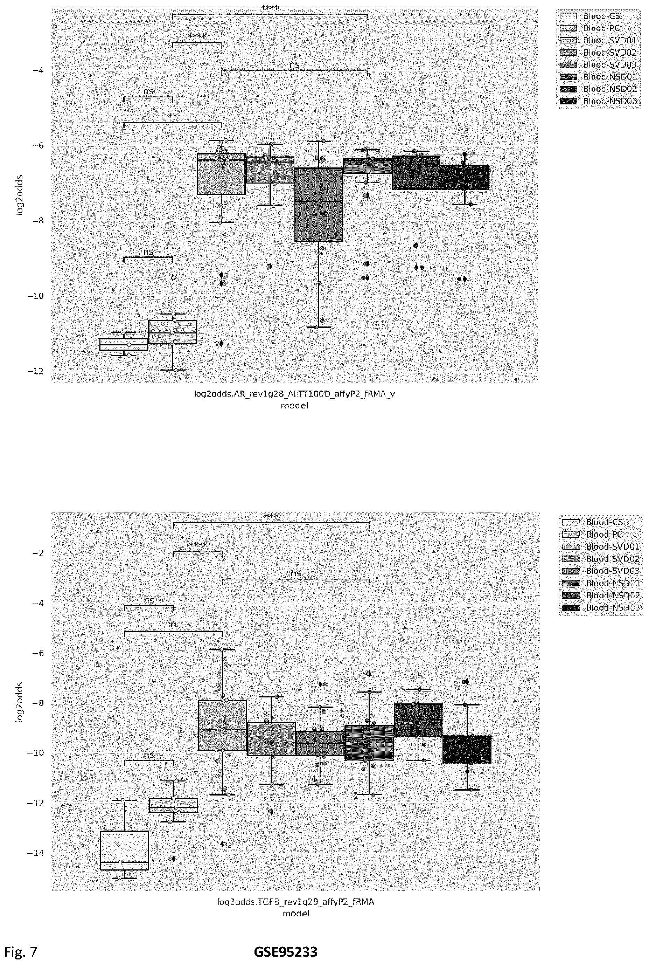

Fig. 7 shows AR signaling pathway activity (top) and TGFbeta signaling pathway activity (bottom) for septic shock patients and healthy control subjects from dataset GSE95233. The data are obtained from whole blood samples from control subjects (CS = healthy control subject; PC = non-septic patient control), septic shock patients (NS = Non-survivors) and septic shock patients (SV = Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

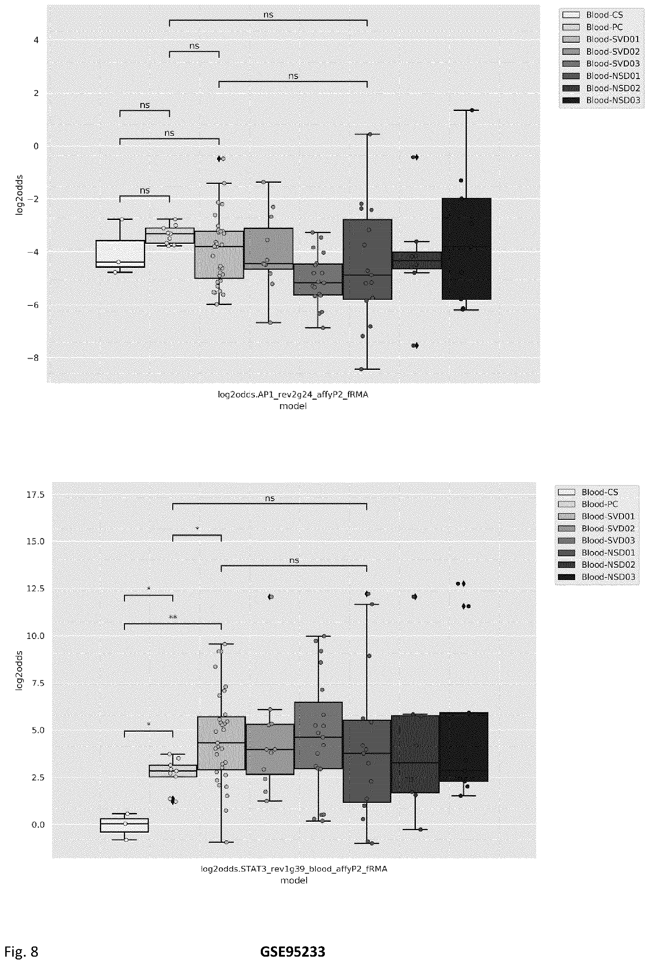

Fig. 8 shows MPK-AP1 signaling pathway activity (top) and JAK-STAT3 signaling pathway activity (bottom) for septic shock patients and healthy control subjects from dataset GSE95233. The data are obtained from whole blood samples from control subjects (CS = healthy control subject; PC = non-septic patient control), septic shock patients (NS = Non-survivors) and septic shock patients (SV = Survivors). The graphs show the log2odds for the respective signaling pathway activity; statistical differences are indicated above the bars, where "ns" (not significant) depicts a p-value of 5.00e^-02 < p <= 1.00e+00, * depicts a p value of 1.00e^-02 < p <= 5.00e-02, ** depicts a p value of 1.00e^-03 < p <= 1.00e-02, *** depicts a p value of 1.00e^-04 < p <= 1.00e-03, and **** depicts a p value of p <= 1.00e-04.

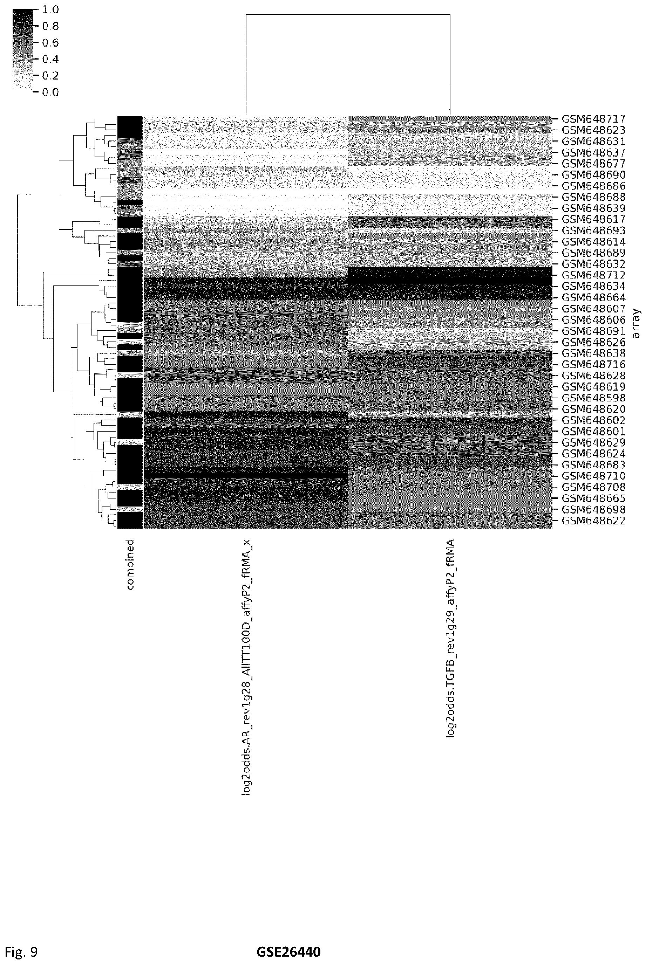

Fig. 9 shows a clustering diagram for the individual samples in dataset GSE26440 based on the AR and TGFbeta signaling pathways. The greyscale coding represents a logarithmic scale for the individual pathway scores. Hierarchical clustering was used. The color coding on the left side depicts: black = septic shock patient (survivor); light grey = septic shock patient (non-survivor); medium grey = normal control; dark grey = control survivor.

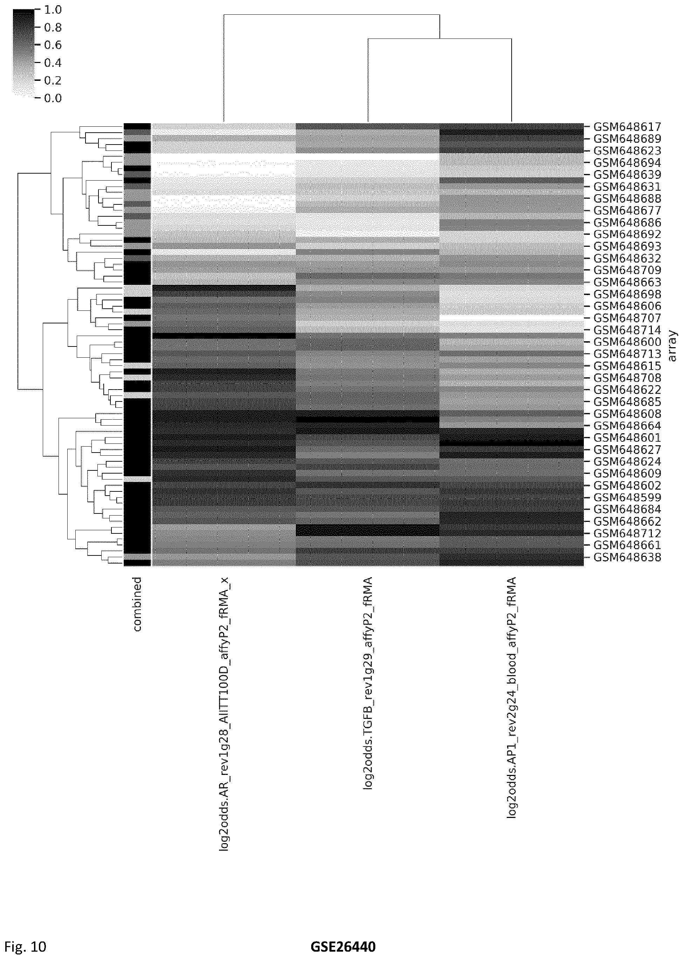

Fig. 10 shows a clustering diagram for the individual samples in dataset GSE26440 based on the AR, TGFbeta and MAPK-AP1 signaling pathways. The greyscale coding represents a logarithmic scale for the individual pathway scores. Hierarchical clustering was used. The color coding on the left side depicts: black = septic shock patient (survivor); light grey = septic shock patient (non-survivor); medium grey = normal control; dark grey = control survivor.

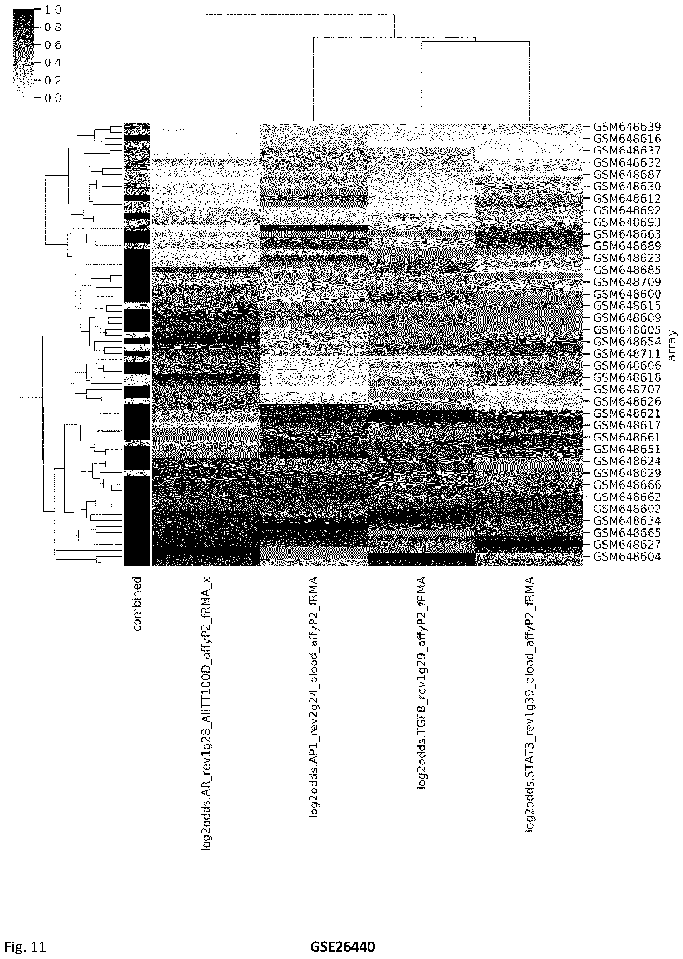

Fig. 11 shows a clustering diagram for the individual samples in dataset GSE26440 based on the AR, TGFbeta, MAPK-AP1 and JAK-STAT3 signaling pathways. The greyscale coding represents a logarithmic scale for the individual pathway scores. Hierarchical clustering was used. The color coding on the left side depicts: black = septic shock patient (survivor); light grey = septic shock patient (non-survivor); medium grey = normal control; dark grey = control survivor.

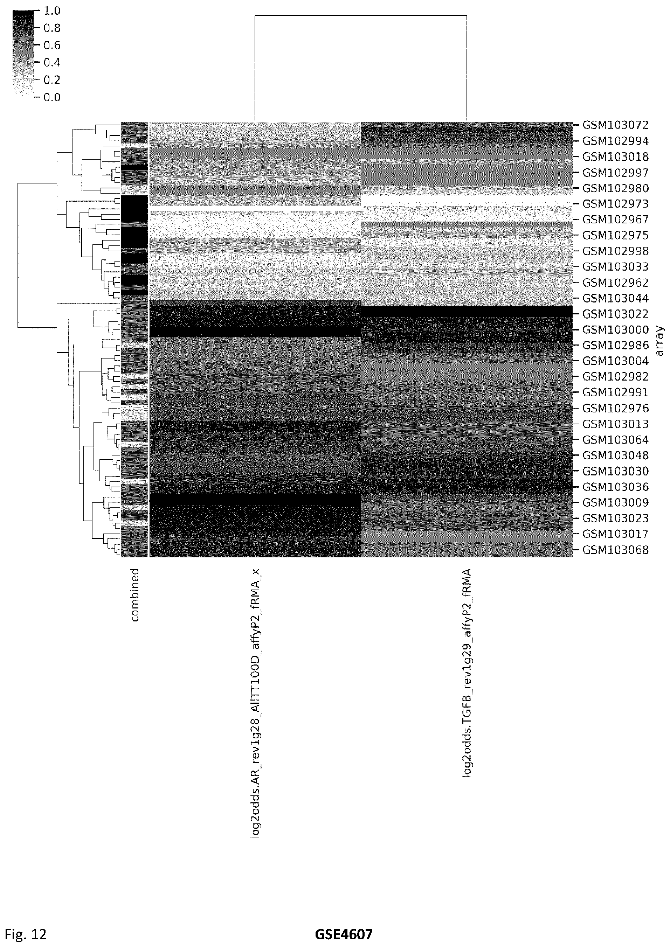

Fig. 12 shows a clustering diagram for the individual samples in dataset GSE4607 based on the AR and TGFbeta signaling pathways. The greyscale coding represents a logarithmic scale for the individual pathway scores. Hierarchical clustering was used. The color coding on the left side depicts: black = control; light grey = septic shock patient (non-survivor); dark grey = septic shock patient (survivor).

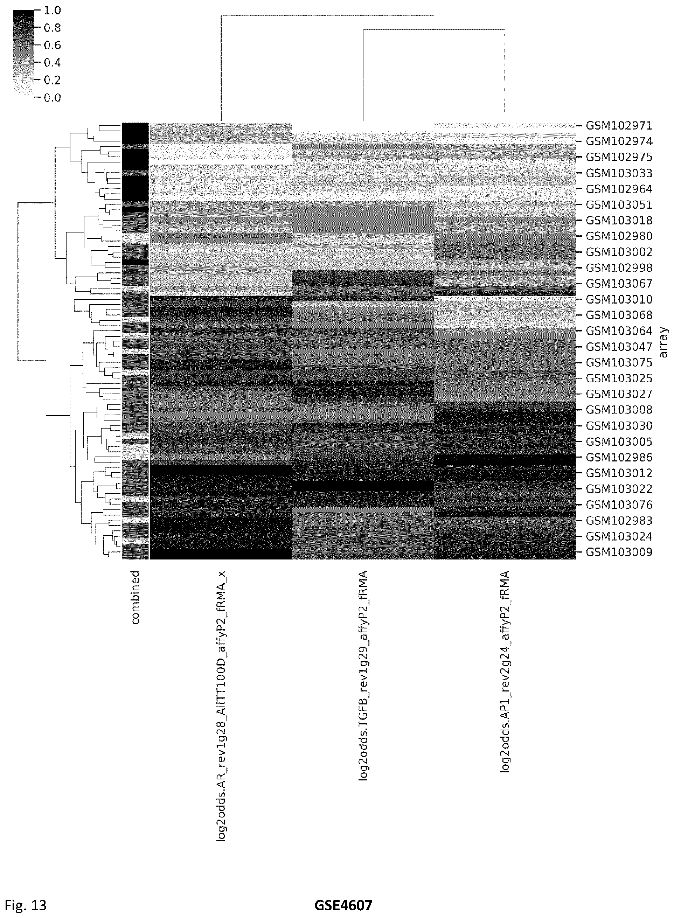

Fig. 13 shows a clustering diagram for the individual samples in dataset GSE4607 based on the AR, TGFbeta and MAPK-AP1 signaling pathways. The greyscale coding represents a logarithmic scale for the individual pathway scores. Hierarchical clustering was used. The color coding on the left side depicts: black = control; light grey = septic shock patient (non-survivor); dark grey = septic shock patient (survivor).

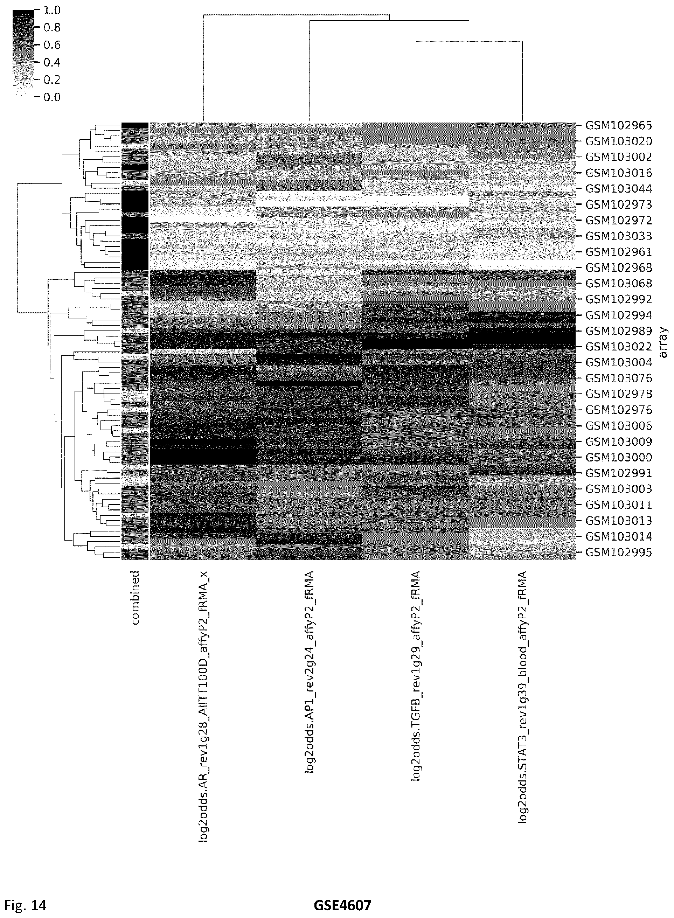

Fig. 14 shows a clustering diagram for the individual samples in dataset GSE4607 based on the AR, TGFbeta, MAPK-AP1 and JAK-STAT3 signaling pathways. The greyscale coding represents a logarithmic scale for the individual pathway scores. Hierarchical clustering was used. The color coding on the left side depicts: black = control; light grey = septic shock patient (non-survivor); dark grey = septic shock patient (survivor).

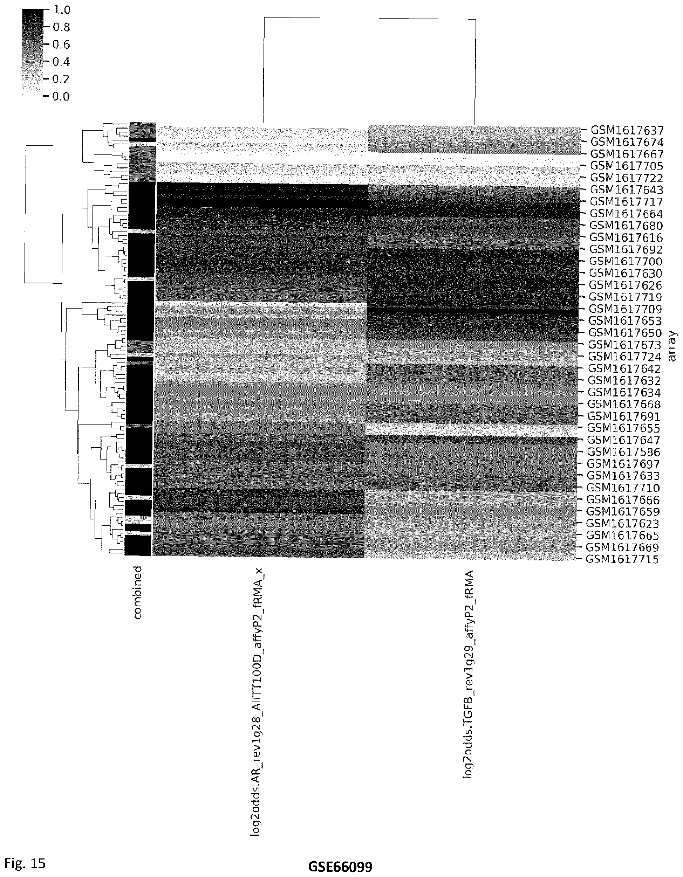

Fig. 15 shows a clustering diagram for the individual samples in dataset GSE66099 based on the AR and TGFbeta signaling pathways. The greyscale coding represents a logarithmic scale for the individual pathway scores. Hierarchical clustering was used. The color coding on the left side depicts: black = septic shock patient; light grey = septic patient; dark grey = control subject.

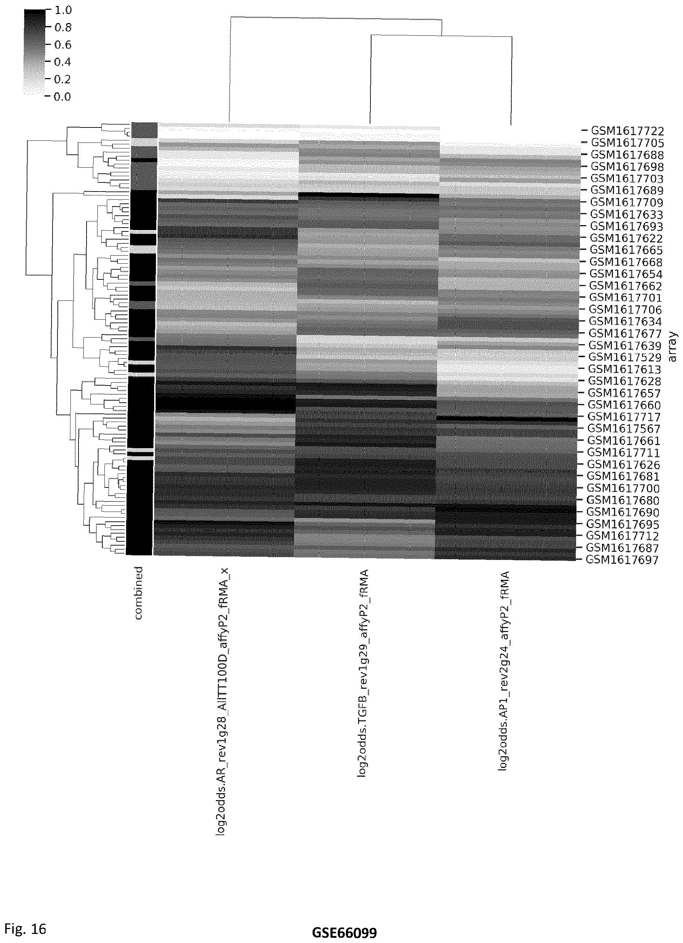

Fig. 16 shows a clustering diagram for the individual samples in dataset GSE66099 based on the AR, TGFbeta and MAPK-AP1 signaling pathways. The greyscale coding represents a logarithmic scale for the individual pathway scores. Hierarchical clustering was used. The color coding on the left side depicts: black = septic shock patient; light grey = septic patient; dark grey = control subject.

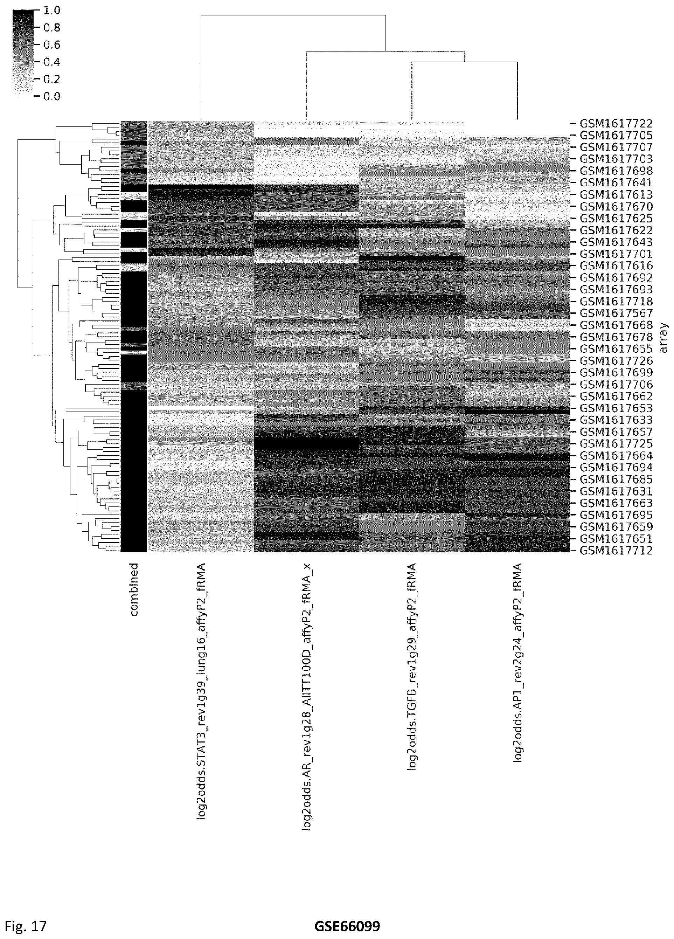

Fig. 17 shows a clustering diagram for the individual samples in dataset GSE66099 based on the AR, TGFbeta, MAPK-AP1 and JAK-STAT3 signaling pathways. The greyscale coding represents a logarithmic scale for the individual pathway scores. Hierarchical clustering was used. The color coding on the left side depicts: black = septic shock patient; light grey = septic patient; dark grey = control subject.

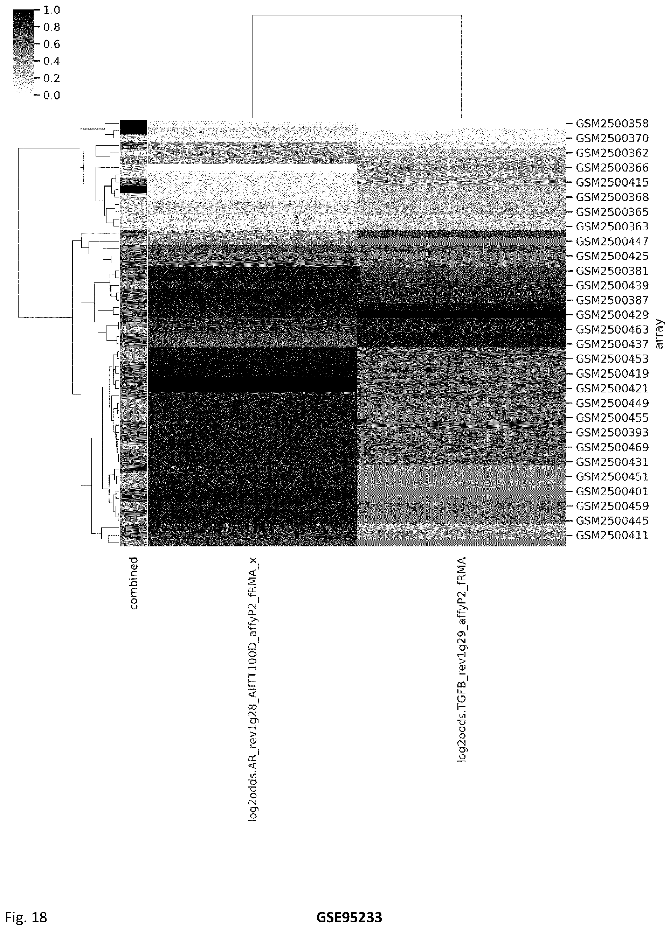

Fig. 18 shows a clustering diagram for the individual samples in dataset GSE95233 based on the AR and TGFbeta signaling pathways. The greyscale coding represents a logarithmic scale for the individual pathway scores. Hierarchical clustering was used. The color coding on the left side depicts: black = septic shock patient; light grey = septic patient; dark grey = control subject.

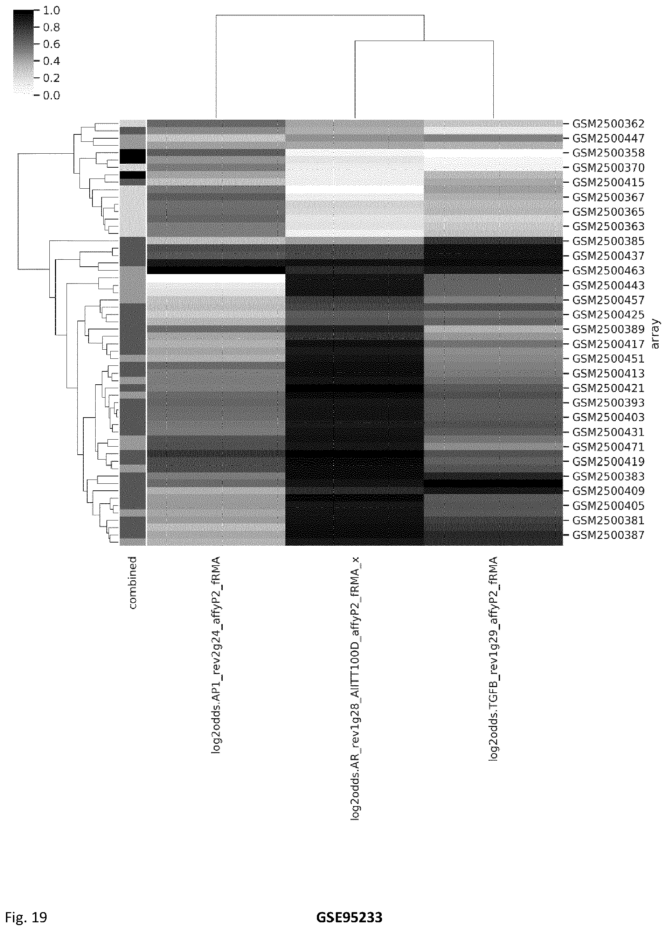

Fig. 19 shows a clustering diagram for the individual samples in dataset GSE95233 based on the AR, TGFbeta and MAPK-AP1 signaling pathways. The greyscale coding represents a logarithmic scale for the individual pathway scores. Hierarchical clustering was used. The color coding on the left side depicts: black = septic shock patient; light grey = septic patient; dark grey = control subject.

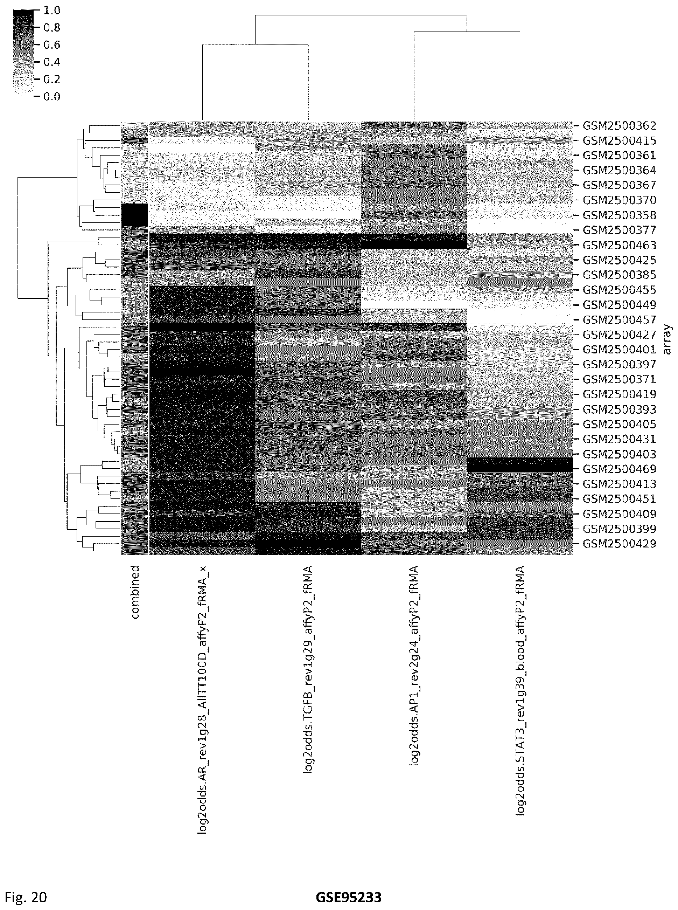

Fig. 20 shows a clustering diagram for the individual samples in dataset GSE95233 based on the AR, TGFbeta, MAPK-AP1 and JAK-STAT3 signaling pathways. The greyscale coding represents a logarithmic scale for the individual pathway scores. Hierarchical clustering was used. The color coding on the left side depicts: black = blood control; light grey = control survivor; medium grey = non-survivor day 1; dark grey = survivor day 1.

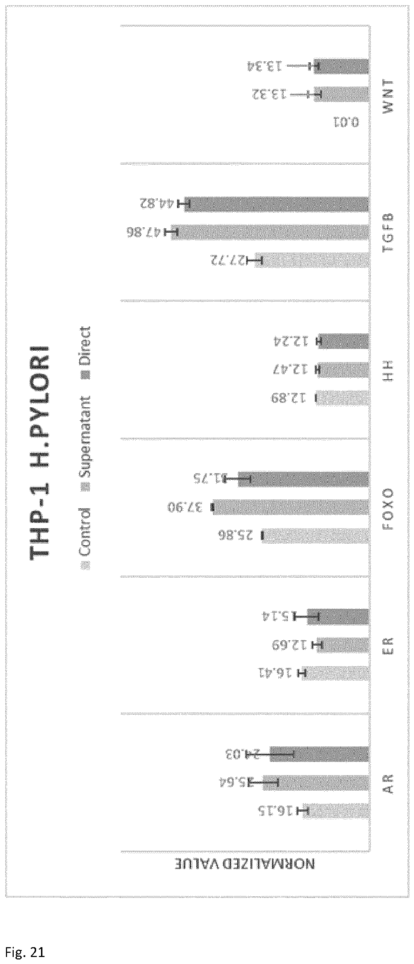

Fig. 21 depicts the pathway activities obtained from isolated THP-1 cells. THP-1 cells were incubated with H. pylori bacteria supernatant, directly incubated with H. pylori bacteria and compared with control THP-1 cells. Activities of the AR, ER, FOXO Hedgehog and TGFbeta pathways were determined and relative values are plotted.

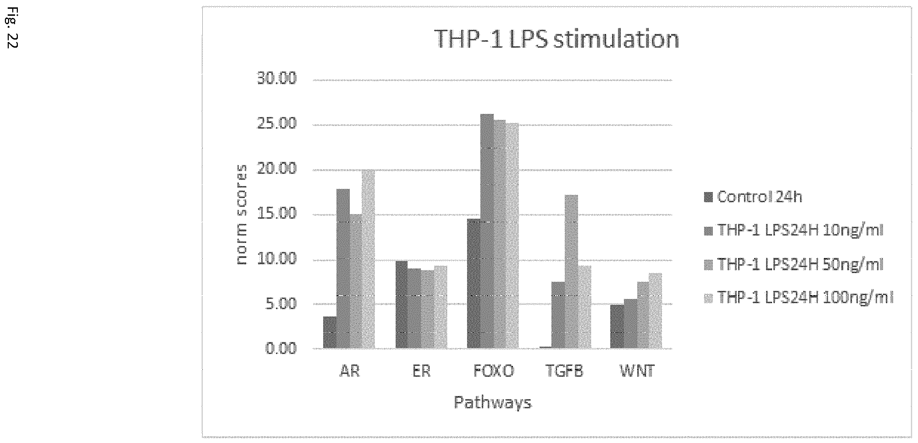

Fig. 22 depicts the pathway activities obtained from isolated THP-1 cells. THP-1 cells were incubated with different concentrations of the bacterial product lipopolysaccharide (LPS) and compared with control THP-1 cells. Activities of the AR, ER, FOXO Hedgehog and TGFbeta pathways were determined and relative values are plotted.

## DETAILED DESCRIPTION OF EMBODIMENTS

The following examples merely illustrate particularly preferred methods and selected aspects in connection therewith. The teaching provided herein may be used for constructing several tests and/or kits, e.g., to detect, predict and/or diagnose the functional status of one or more blood samples. Furthermore, upon using methods as described herein drug prescription can advantageously be guided, drug response prediction and monitoring of drug efficacy (and/or adverse effects) can be made. The following examples are not to be construed as limiting the scope of the present invention.

**Example 1 - Methods and sample description**

Using the Gene Expression Omnibus (GEO) database (https://www.ncbi.nlm.nih.gov/gds/) Affymetrix HG-U133Plus2.0 data from samples from clinical and preclinical studies in which whole blood samples (GSE26440, GSE4607, GSE66099, GSE95233, for more information about sample type and preparation see Table 1) were used. We used the pathway analysis to determine the signal transduction pathway activities (AR, ER, PR, GR, HH, Notch, TGFbeta, WNT, JAK-STAT1/2, JAK-STAT3, NFkB, PI3K, MAPK). For the hierarchical clustering we used the clustering tool Seaborn clustermap.

For analysis public Affymetrix U133P2.0 data were used from the GEO database (GSE26440, GSE4607, GSE66099, GSE95233, for more information about sample type and preparation see Table 1). Pathway analysis of datasets GSE26440, GSE4607, GSE66099 and GSE95233 showed significant differences in multiple pathways including AR and TGFBeta pathway activity using Mann-Whitney-Wilcoxon two-sided test between normal (healthy) control subjects and septic shock subjects (for pathways and p values see Figures 1-8).

Using a combination of significant pathways, we could identify/diagnose the sepsis subjects from the controls. Furthermore, based on the AR and or TGFBeta pathway activity a computational model was made to calculate a risk score with respect to the risk to die and survive from sepsis. With hierarchical clustering we identified samples which clustering near control/healthy people, which are more likely to survive.

**Table 1:** Sample type and preparation information per dataset.

| GEO Dataset | Patients original in dataset | Patients | Method |
|---|---|---|---|
| **GSE26440** | 76 | Children | blood samples were obtained within 24 hours |
|  | (duplicates removed for analysis) | <=10 years | of initial presentation to the paediatric intensive care unit with septic shock. **Total RNA** was isolated from **whole blood samples** using the **PaxGene™ blood RNA system.** |
| **GSE4607** | 83 | Children < 10 years | blood samples (for RNA and serum isolation) were obtained within 24 hours of admission to the PICU, heretofore referred to as "Day 1" of septic shock. Total RNA was isolated from **whole blood samples using the PaxGene™ Blood RNA System** (PreAnalytiX, Qiagen/Becton Dickson, Valencia, CA Patients meeting criteria for "sepsis" or "severe sepsis" were placed in the categories of SIRS and septic shock, respectively, for study purposes. |
| **GSE66099** (unique patients from GSE4607, GSE8121, GSE9692, GSE13904, GSE26378, and GSE26440) | 128 (duplicates removed for analysis) | Patients from 5 days to 18 years old. | Consist of dataset; GSE4607, GSE8121, GSE9692, GSE13904, GSE26378, and GSE26440, data was renormalized again. **All datasets use the PaxGene™ Blood RNA System and whole blood as input** |
| **GSE95233** | 125 | 53-80 years | The first **whole blood sample** (EDTA tubes) was collected at the onset of shock (i.e., within 30 min after the beginning of vasoactive treatment, D0) Total RNA was extracted with **PAXgene™ Blood RNA kit.** |

22

*Example 2: Computational models to calculate a risk score*

Furthermore, we were able to classify low, medium and high-risk sepsis subjects, with respect to the risk to die from sepsis. We used the computational model-based interpretation of multiple signaling pathway activity scores to classify the low, medium and high risk sepsis subjects.

To construct a linear model for interpretation of pathway activity scores we assessed the pathway activities in healthy people, by calculating the average pathway activity with 1 and 2 Standard Deviation (SD). When a pathway activity falls outside the boundaries of 2SD of the normal healthy, we consider this an abnormally active pathway, which means in the model 1 point. Optionally another threshold, such as 3SD of the mean, can be used. Adding up the points generates a cumulative abnormal pathway activity score, which directly determines the likelihood of the risk.

Other computational models to calculate a risk score can be Bayesian models, centroid-based models etc.

*Example 3: Linear model using calibration and validation set*

For this, we used dataset GSE26440 as training set model and validated the model with dataset GSE4607. For both the AR and TGFBeta pathway 2SD above the mean pathway activity scores measured in the healthy control population was used for the classification model, this same value was then applied to the independent validation dataset GSE4607. When both AR and TGFBeta were 2SD higher than the control samples, the sepsis subjects were classified as high-risk (2 points). When either AR or TGFBeta were 2SD higher than the control, subjects were classified as medium-risk (1 point) and less than 2 SD difference was classified as low-risk (0 points). See table 2 for the determined means, standard deviations and 2SD top boundary for the pathways.

For the prognostic model, low, medium and high risk groups are identified for subject stratification. In the medium group one of the pathways is upregulated whereas in the high group both pathways are upregulated.

In table 3 the performance of the prognostic model is shown. For the GSE 26440 dataset (n=76, non survivor 10% (n=8), survivor 68% (n=51), Control 22% (n=17)) we could classify of the non survivors group, 3 as high risk, 5 as medium risk and 0 as low risk. For the validation set GSE 4607 (n=83, non survivor 17% (n=14), survivor 65% (n=54) and control 18% (n=15)) we could classify of the non survivors group 10 as high, 2 as medium and 2 as low using the model described above.

In addition, the combined pathway sum score of AR and TGFBeta can also be used for the prognostic marker, in which high risk is classified (1 point) as the combined AR and TGFBeta pathway score were 2SD higher than the control samples. When the combined AR and TGFBeta score were less than 2 SD difference compared to control, the samples are classified as low-risk (0 points) (data not shown).

For the other datasets (GSE66099, GSE95233 and GSE57064) we also see samples with a low AR and/or TGFBeta pathway activity. However, we lack survival data to prove that these subjects have a higher change to survive from sepsis.

Table 2: Mean values, standard deviations and 2SD top boundary of the activity of AR TGFbeta and combined based on GSE26440.

| Pathway | Mean | Standard deviation | 2SD Top boundary |
|---|---|---|---|
| AR | 18.0 | 2.6 | 23.3 |
| TGFBeta | 13.1 | 2.6 | 18.4 |
| AR+TGFBeta combined | 31.2 | 4.4 | 40.0 |

Table 3: Model for the classification of low, medium and high-risk sepsis subjects that are likely to die. (n=number of samples). SD based on top boundary using control group. Scoring < 2SD of AR or TGFBeta -> Low, >2 SD of only AR or TGFBeta -> Medium, >2 SD of AR and TGFBeta -> High.

| Model (2SD of AR, TGFBeta) | Training GSE 26440 (n=76, non survivor (8) survivor (51) control (17)) | | Validation GSE 4607 (n=83, non survivor 17 % (14) survivor 65 % (54) control 18% (15)) | |
|---|---|---|---|---|
| | Prediction NS and S | | Prediction NS and S | |
| High risk (AR + TGFBeta high) | 38% (3/8) NS 43% (22/51) S | non survivor (3) survivor (22) control (0) | 71% (10/14) NS 57% (31/54) S | non survivor (10) survivor (31) control (0) |

(continued)

| Model (2SD of AR, TGFBeta) | Training GSE 26440 (n=76, non survivor (8) survivor (51) control (17)) | | Validation GSE 4607 (n=83, non survivor 17 % (14) survivor 65 % (54) control 18% (15)) | |
|---|---|---|---|---|
| Medium risk (ARor TGFBeta high) | 62% (5/8) NS 35% (18/51) S | non survivor (5) survivor (18) control (2) | 14% (2/14) NS 28% (15/54) S | non survivor (2) survivor (15) control (0) |
| Low risk (AR or TGFBeta low) | 0% (0/8) NS 22% (11/51) S | non survivor (0) survivor (11) control (15) | 14% (2/14) NS 64% (9/14) S | non survivor (2) survivor (9) control (15) |

*Example 4: Linear model 2 - using top boundaries per dataset*

Due to the differences between tests and sample taking it is probably more specific to determine top boundaries per dataset. In table 4 the Mean values, standard deviations and SD top boundaries of the activity of AR of GSE26440 and GSE4607 are listed. In table 5, the above described linear model is used but in this example a 2 SD top boundary is used based on each separate dataset. All non-survivals are placed in the medium and high-risk group. The control samples are only located in the low group and could be used as a diagnostic marker.

Table 4: Mean values, standard deviations and SD top boundaries of the activity of AR of GSE26440 and GSE4607

| Dataset | Mean | Standard deviation | 1SD Top boundary | 2SD Top boundary | 3SD Top boundary |
|---|---|---|---|---|---|
| GSE26440 | 18.0 | 2.6 | 20.6 | 23.3 | 25.9 |
| GSE4607 | 17.8 | 1.8 | 19.6 | 21.4 | 23.3 |

Table 5: Model for the classification of Low risk, medium risk and high risk sepsis subjects that are likely to die based on AR + TGFBeta. SD based on top boundary using control group. Scoring < 2SD of AR or TGFBeta -> Low, >2 SD of only AR or TGFBeta -> Medium, >2 SD of AR and TGFBeta -> High. n=number of samples).

| Model 2xSD | GSE 26440 (n=76, non survivor (8) survivor (51) control (17)) | | GSE 4607 (n=83, non survivor 17 % (14) survivor 65 % (54) control 18% (15)) | |
|---|---|---|---|---|
| | Prediction NS and S | | Prediction NS and S | |
| High risk (AR + TGFBeta high) | 38% (3/8) NS 43% (22/51) S | non survivor (3) survivor (22) control (0) | 79% (11/14) NS 69% (37/54) S | non survivor (11) survivor (37) control (0) |
| Medium risk (AR or TGFBeta high) | 62% (5/8) NS 35% (18/51) S | non survivor (5) survivor (18) control (2) | 21% (3/14) NS 15% (8/54) S | non survivor (3) survivor (8) control (0) |
| Low risk (AR or TGFBeta low) | 0% (0/8) NS 22% (11/51) S | non survivor (0) survivor (11) control (15) | 0% (0/14) NS 17% (9/54) S | non survivor (0) survivor (9) control (15) |

*Example 5: Linear model 3 - Using only AR pathway and top boundaries per dataset*

The same principle as described above is used in this example, only a 2DS top boundary is used and the model is based on only the AR pathway. The risk groups are in this case low or high risk of dying from sepsis. The performance of the model can be found in Table 6. The control samples are only located in the low group and could be used as a diagnostic marker.

Table 6: Prognostic model for the classification of Low and high-risk sepsis subjects that are likely to die and survive. (n=number of samples). SD based on top boundary using control group. Scoring < 2SD of AR -> Low, >2 SD of AR -> High.

| Model (2SD of AR) | GSE 26440 (n=76, non survivor (8) survivor (51) control (17)) | | GSE 4607 (n=83, non survivor 17 % (14) survivor 65 % (54) control 18% (15)) | |
|---|---|---|---|---|
| | Prediction NS and S | | Prediction NS and S | |
| High (AR) | **100% (8/8) NS** 61% (31/51) S | non survivor (8) survivor (31) control (1) | **93% (13/14) NS** 72% (39/54) S | non survivor (13) survivor (39) control (0) |
| Low (AR) | 0% (0/8) NS **39% (20/51) S** | non survivor (0) survivor (20) control (16) | 7% (1/14) NS **28% (15/54) S** | non survivor (1) survivor (15) control (15) |

*Example 6: Clustering methods:*

We used hierarchical clustering (seaborn clustermap) to determine if it was possible to classify subjects based on their pathway activity. We selected the significant models between the control group and sepsis groups for the pathways AR, TGFBeta, JAK-STAT3 and MAPK-AP1.

For dataset GSE4607, several sepsis samples are clustered near the control group (orange). These subjects probably have a higher chance on survival. These samples are also clustered in the low risk group in the model described above which was based on the AR and TGFBeta. However, a clear distinction between the Septic Shock survivor and non survivors is not shown.

*Example 7: Cell stimulation with H. Pylori*

To investigate whether bacteria or bacterial products could induce the same pathway activities as observed in patients with sepsis, in vitro experiments were performed in which either bacteria or the bacterial product LPS was added to monocytic cells from a cell line, as a model system for monocytes in blood.

Porphyromonas gingivalis (ATCC, 33277) bacteria were cultured in an anaerobic culture hood using dehydrated HBI (Oxoid, CM1032) using manufactures instructions.

THP-1 cells were cultured in 6-wells plates with a density of 4x105 cells/well for 48 hours. After 48-hour cell seeding, cells were washed with PBS and treated with either direct bacteria of 1:100 MOI or using the 20% 'supernatant' of the bacterial culture at 37°C 5%CO2 for 4 hours. Cells were exposed to bacteria with a MOI of 1:100, the 20% 'supernatant' was prepared by filtering the overnight bacteria culture was fusing 0.2 uM filter to remove whole bacteria and hereafter diluted in the cell culture media to a 20% concentration. After the 48-hour cell seeding, cells were washed with PBS and treated with either direct bacteria of 1:100 MOI or using the 20% 'supernatant' of the bacterial culture at 37°C 5%CO2 for 4 hours. Hereafter, cells were washed with PBS and lysed in RNeasy mini kit lysis buffer (Qiagen, Cat No./ID: 74104) and stored at -80°C until further processing. RNA was extracted using the RNeasy mini kit (Qiagen, 74104). qPCR was performed using the Philips Research OncoSignal platform.

To determine whether the Helicobacter bacteria have a bacteria-specific effect on the pathways activities of THP-1 cells, qRT-PCR was performed. Cells were either treated with direct bacteria or the growth media of the bacteria culture. As shown in Figure 21, the pathway activity was increased for the AR, FOXO, TGFbeta and the WNT pathway. In the sepsis samples however, we did not detected a significant difference in the FOXO and WNT pathways, which could be due to the fact that monocytes only consists of 4-8% of the blood composition, and other blood cell types also play an important role.

*Example 8: Cell stimulation with LPS*

To study the inflammation process we stimulated the monocytic THP-1 cells (ATCC® TIB-202™) using 3 different concentrations of LPS originated from E.coli (0ng/ml, 10ng/ml, 50ng/ml and 100ng/ml) into culture medium (DMEM, supplemented with 10% FBS, 1% glutamax and 1% pen strep at 37°C 5%CO2.) of THP-1 for 24 hours. After stimulation cells were harvested and RNA was extracted using the RNeasy mini kit (Qiagen, 74104). qPCR was performed using the Philips Research OncoSignal platform. As shown in Figure 22, the pathway activity was increased for the pathways AR, FOXO, TGFBeta and WNT in the LPS stimulated cells. The activation of the AR and TGFBeta pathways was also seen in sepsis samples confirms the role of these pathways in inflammation. In the sepsis samples however, we did not detected a significant difference in the FOXO and WNT pathways, which could be due to the fact that monocytes only consists of 4-8% of the blood composition, and other blood cell types also play an important role.

**References:**

[1] N. L. Stanski and H. R. Wong, "Prognostic and predictive enrichment in sepsis," Nature Reviews Nephrology. Nature Publishing Group, 01-Jan-2019.

[2] "Recommendations | Sepsis: recognition, diagnosis and early management | Guidance | NICE."

[3] N. K. Patil, J. K. Bohannon, and E. R. Sherwood, "Immunotherapy: A promising approach to reverse sepsis-induced immunosuppression.," Pharmacol. Res., vol. 111, pp. 688-702, 2016.

[4] R. S. Hotchkiss, G. Monneret, and D. Payen, "Sepsis-induced immunosuppression: from cellular dysfunctions to immunotherapy.," Nat. Rev. Immunol., vol. 13, no. 12, pp. 862-74, Dec. 2013.

[5] M. R. Gubbels Bupp and T. N. Jorgensen, "Androgen-Induced Immunosuppression," Front. Immunol., vol. 9, p. 794, Apr. 2018.

[6] A. Trigunaite and J. Dimo, "Suppressive effects of androgens on the immune system," Cell. Immunol., vol. 294, no. 2, pp. 87-94, Apr. 2015.

[7] von Dipl Biochem Daniela Röll, "Androgen-regulation of sepsis response: Beneficial role of androgen receptor antagonists."

[8] F. Fattahi and P. A. Ward, "Understanding Immunosuppression after Sepsis," Nat. Rev. Mol. Cell Biol, vol. 42, pp. 51-65, 2017.

[9] A. Roquilly et al., "Local Modulation of Antigen-Presenting Cell Development after Resolution of Pneumonia Induces Long-Term Susceptibility to Secondary Infections," Immunity, vol. 47, no. 1, pp. 135-147.e5, Jul. 2017.

[10] M. Cecconi et al., "Sepsis and septic shock", Lancet 2018; 392: 75-87

**Claims**

1. A method for determining a functional status of a blood sample, based on RNA extracted from the blood sample, the method comprising the steps of:

   - determining or receiving the result of a determination of the expression level of three or more target genes of the AR pathway;
   - determining the AR signaling pathway activity, based on the determined expression levels of said three or more target genes of the AR signaling pathway;
   - determining the functional status of the blood sample based at least on the determined AR signaling pathway activity, wherein said functional status of said blood sample is being determined as having the determined AR signaling pathway activity, wherein said blood sample is obtained from a subject with sepsis or obtained from a subject suspected to have sepsis or an subject at risk of developing sepsis.

2. Method according to claim 1, wherein said method further comprises, determining or receiving the result of a determination of the expression level of three or more target genes of the TGFbeta pathway, determining the TGFbeta signaling pathway activity based on the determined expression levels of said three or more target genes of the TGFbeta signaling pathway, and wherein said functional status of said blood sample is further based on the determined TGFbeta signaling pathway activity, wherein said functional status is further being determined as having the determined TGFbeta signaling pathway activity.

3. Method according to any one of the preceding claims, wherein said method further comprises:

   determining or receiving the result of a determination of the expression level of three or more target genes of the MAPK-AP1 signaling pathway, and determining the MAPK-AP1 signaling pathway activity based on said expression levels of the three or more target genes of the MAPK-AP1 signaling pathway, and/or determining or receiving the result of a determination of the expression level of three or more target genes of the JAK-STAT3 signaling pathway, and determining the JAK-STAT3 signaling pathway activity based on said expression levels of the three or more target genes of the JAK-STAT3 signaling pathway,

and wherein said functional status of said blood sample is further based on the determined MAPK-AP1 signaling pathway activity and/or JAK-STAT3 signaling pathway activity , wherein said functional status is further being determined as having the determined MAPK-AP1 signaling pathway activity, and/or wherein said functional status is further being determined as having the determined JAK-STAT3 signaling pathway activity.

4. Method according to any one of the preceding claims wherein said determining the expression level of three or more target genes of the AR signaling pathway, the TGFbeta signaling pathway, the MAPK-AP1 signaling pathway and/or the JAK-STAT3 signaling pathway comprises:

   determining the expression level of three or more target genes of the AR signaling pathway selected from the list consisting of: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, and/or;
   determining the expression level of three or more target genes of the TGFbeta signaling pathway comprises determining the expression level of three or more target genes selected from the list consisting of ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, and/or;
   determining the expression level of three or more target genes of the MAPK-AP1 signaling pathway comprises determining the expression level of three or more target genes selected from the list consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, and/or;
   determining the expression level of three or more target genes of the JAK-STAT3 signaling pathway comprises determining the expression level of three or more target genes selected from the list consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1.

5. Method according to any one of the preceding claims, wherein said method further comprises the step of diagnosing the subject from which the blood sample has been obtained, wherein said subject is diagnosed to have sepsis or wherein said subject is diagnosed to not have sepsis based on:

   - a clinical parameter, and
   - the functional status of the blood sample,

   the method further comprising comparing the functional status of the blood sample of the subject to at least one functional status of a blood sample obtained from a healthy or non-septic control subject, preferably wherein the subject is diagnosed to have sepsis if the functional status of the blood sample comprises an AR signaling pathway activity which AR signaling pathway activity is determined to be higher than the AR signaling pathway activity determined in the control blood sample obtained from a healthy or non-septic control and the subject from which the blood sample has been obtained has at least one clinical parameter associated with sepsis.

6. Method according to any one of the preceding claims, wherein said functional status of the blood sample is used in predicting the mortality risk for the subject from which the blood sample has been obtained, wherein said prediction is based on a comparison of the functional status of the blood sample of the subject with a plurality of reference functional statuses of the blood samples obtained from reference subjects, wherein said plurality of reference functional statuses of the blood samples obtained from reference subjects comprises at least one functional statuses of blood sample obtained from subject with sepsis which is a non-survivor and at least one functional statuses of blood samples obtained from subject with sepsis which is a survivor, and optionally further comprises at least one functional statuses of blood samples obtained from a healthy or non-septic control subject, wherein the subject from which the blood sample is obtained is confirmed to have sepsis, and wherein a low mortality risk is predicted when the functional status of the blood sample obtained from the subject with sepsis is similar to the at least one functional status of the blood sample obtained from reference subject with sepsis which is a survivor or when the functional status of the blood sample obtained from the subject with sepsis is similar to the at least one functional status of the blood sample obtained from the at least one healthy or non-septic control subject, and

wherein a high mortality risk is predicted when the functional status of the blood sample obtained from the subject with is similar to the at least one functional status of the blood sample obtained from the reference subject with sepsis which is a non-survivor.

7. Method according to claim 6, wherein said comparing of the functional status of the blood sample obtained from the subject with a plurality of functional statuses of the blood samples obtained from control subjects is performed using clustering of the determined pathway activities, preferably by hierarchical clustering.

8. Method according to any one of claims 1 to 4, wherein the subject from which the blood sample has been obtained does not have sepsis, and wherein the functional status of the blood sample is used to determine the risk that the subject will develop sepsis,
the method further comprising comparing the functional status of the blood sample of the subject from which the blood sample has been obtained to at least one functional status of a blood sample obtained from a healthy or non-septic control subject,
preferably wherein the subject from which the blood sample has been obtained is predicted to be at risk to develop sepsis if the functional status of the blood sample comprises an AR signaling pathway activity which AR signaling pathway activity is determined to be higher than the AR signaling pathway activity determined in the control blood sample obtained from a healthy or non-septic control subject.

9. Method according to any one of claims 1 to 4, wherein the subject from which the blood sample has been obtained has recovered from sepsis, and wherein the functional status of the blood sample is used to monitor the risk that the subject will develop a recurrence of sepsis,
the method further comprising comparing the functional status of the blood sample of the subject from which the blood sample has been obtained to at least one functional status of a blood sample obtained from a healthy or non-septic control subject,
preferably wherein the subject from which the blood sample has been obtained is predicted to be at risk to develop a recurrence of sepsis if the functional status of the blood sample comprises an AR signaling pathway activity which AR signaling pathway activity is determined to be higher than the AR signaling pathway activity determined in the control blood sample obtained from a healthy or non-septic control subject.

10. Method according to any one of the preceding claims, wherein the blood sample is a whole blood sample, isolated peripheral blood mononuclear cells (PBMCs), isolated CD4+ cells, isolated CD8+ cells, Regulatory T-cells, mixed CD8+ and T cells, myeloid derived suppressor cells (MDSC), dendritic cells, isolated neutrophils, isolated lymphocytes or isolated monocytes.

11. Method according to any one of the preceding claims, wherein said signaling pathway activity or signaling pathway activities is determined based on evaluating a calibrated mathematical model relating the to the three or more expression levels determined for the pathway or pathways based on the RNA extracted from a blood sample to the activity or activities of the signaling pathway or signaling pathways.

12. A apparatus for determining the functional status of a blood sample, the apparatus comprising a digital processor configured to perform the method according to any one of the preceding claims, comprising an input adapted to receive data indicative of a target gene expression profile for the three or more target genes of the AR signaling pathway, optionally data indicative of a target gene expression profile for the three or more target genes of the TGFbeta signaling pathway and/or the MAPK-AP1 signaling pathway and/or the JAK-STAT3 signaling pathway.

13. Computer program product comprising instructions which, when the program is executed by a computer, cause the computer to carry out a method comprising:

- receiving data indicative of a target gene expression profile for three or more target genes of the AR signaling pathway, optionally further receiving data indicative of the target gene expression levels of three or more target genes of the TGFbeta signaling pathway and/or the MAPK-AP1 signaling pathway and/or the JAK-STAT3 signaling pathway,
- determining the AR signaling pathway activity, and optionally TGFbeta signaling pathway activity and/or the MAPK-AP1 signaling pathway activity and/or the JAK-STAT3 signaling pathway activity based on the determined expression levels of said three or more target genes of the AR signaling pathway and optionally the TGFbeta signaling pathway and/or the MAPK-AP1 signaling pathway and/or the JAK-STAT3 signaling pathway,
- determining the functional status of the blood sample based on the determined AR signaling pathway activity

and optionally TGFbeta signaling pathway activity and/or the MAPK-AP1 signaling pathway activity and/or the JAK-STAT3 signaling pathway activity, wherein said functional status of said blood sample is being determined as having the determined AR signaling pathway activity and optionally the TGFbeta signaling pathway activity and/or the MAPK-AP1 signaling pathway activity and/or the JAK-STAT3 signaling pathway activity, and
- optionally providing a diagnosis or prediction based on the functional status of the blood sample.

14. Kit of parts, comprising primers for inferring activity of one or more cellular signaling pathway(s) by determining the expression levels of one or more set(s) of target genes of the respective cellular signaling pathway(s), wherein the cellular signaling pathway(s) comprise(s) a AR pathway, and optionally further comprises one or more of an TGFbeta pathway, an MAPK-AP1 pathway and a JAK-STAT3 pathway,
wherein the set of target genes of the AR pathway comprises three or more target genes selected from the group comprising: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, and
wherein the set of target genes of the TGFbeta pathway comprises three or more target genes selected from the group comprising: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, and
wherein the set of target genes of the MAPK-AP1 pathway comprises three or more target genes selected from the group comprising: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, and
wherein the set of target genes of the JAK-STAT3 pathway comprises three or more target genes selected from the group comprising: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, optionally the kit further comprising the apparatus according to claim 12 and/or the computer program product of claim 13.

15. A method for in vivo or ex vitro diagnosing or prognosticating whether a subject has sepsis, has septic shock or has a high mortality risk as a result of sepsis using a kit, the kit comprising primers for inferring activity of one or more cellular signaling pathway(s) by determining the expression levels of one or more set(s) of target genes of the respective cellular signaling pathway(s), wherein the cellular signaling pathway(s) comprise(s) a AR pathway, and optionally further comprises one or more of an TGFbeta pathway, an MAPK-AP1 pathway and a JAK-STAT3 pathway, wherein the set of target genes of the AR pathway comprises three or more target genes selected from the group comprising: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, and
wherein the set of target genes of the TGFbeta pathway comprises three or more target genes selected from the group comprising: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, and
wherein the set of target genes of the MAPK-AP1 pathway comprises three or more target genes selected from the group comprising: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, and
wherein the set of target genes of the JAK-STAT3 pathway comprises three or more target genes selected from the group comprising: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1.

Fig. 1                                    GSE26440

Fig. 2                                    GSE26440

Fig. 3                                                    GSE4607

Fig. 4          **GSE4607**

Fig. 5                                 **GSE66099**

Fig. 6                              GSE66099

Fig. 7                                    **GSE95233**

Fig. 8                                    GSE95233

Fig. 9                                        **GSE26440**

Fig. 10                                    **GSE26440**

Fig. 11

GSE26440

EP 3 882 363 A1

Fig. 12

GSE4607

41

Fig. 13                                    **GSE4607**

EP 3 882 363 A1

Fig. 14                    GSE4607

43

Fig. 15                                             GSE66099

Fig. 16

GSE66099

Fig. 17                                        GSE66099

Fig. 18                                  **GSE95233**

Fig. 19 GSE95233

Fig. 20                                        **GSE95233**

Fig. 21

Fig. 22

Europäisches
Patentamt

European
Patent Office

Office européen
des brevets

# EUROPEAN SEARCH REPORT

Application Number

EP 20 16 3627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANJA VAN DE STOLPE ET AL: "Enabling precision medicine by unravelling disease pathophysiology: quantifying signal transduction pathway activity across cell and tissue types", SCIENTIFIC REPORTS, vol. 9, no. 1, 7 February 2019 (2019-02-07), XP055646064, DOI: 10.1038/s41598-018-38179-x * the whole document * | 1-15 | INV. C12Q1/6883 |
| X,D | WO 2013/011479 A2 (KONINKL PHILIPS ELECTRONICS NV [NL] ET AL.) 24 January 2013 (2013-01-24) * the whole document * | 1-15 | |
| X,D | WO 2014/102668 A2 (KONINKL PHILIPS NV [NL]) 3 July 2014 (2014-07-03) * the whole document * | 1-15 | |
| X | LEO MCHUGH ET AL: "A Molecular Host Response Assay to Discriminate Between Sepsis and Infection- Negative Systemic Inflammation in Critically Ill Patients: Discovery and Validation in Independent Cohorts", PLOS MEDICINE, vol. 12, no. 12, 8 December 2015 (2015-12-08), page e1001916, XP055336105, DOI: 10.1371/journal.pmed.1001916 * the whole document * | 1-15 | TECHNICAL FIELDS SEARCHED (IPC) C12Q |
| X | US 2009/203534 A1 (HOSSAIN HAMID [DE] ET AL) 13 August 2009 (2009-08-13) * paragraph [0029] - paragraph [0048] * * paragraph [0085] - paragraph [0105]; claims 21-37; tables 1,6,7 * | 1-15 | |

-/--

The present search report has been drawn up for all claims

| Place of search | Date of completion of the search | Examiner |
|---|---|---|
| Munich | 4 September 2020 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| | Europäisches Patentamt European Patent Office Office européen des brevets | **EUROPEAN SEARCH REPORT** | Application Number EP 20 16 3627 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/068623 A1 (KONINKLIJKE PHILIPS NV [NL]) 11 April 2019 (2019-04-11) * page 16, line 23 - page 27, line 32; claims 1-15 * ----- | 1-15 | |

TECHNICAL FIELDS SEARCHED (IPC)

The present search report has been drawn up for all claims

| Place of search | Date of completion of the search | Examiner |
|---|---|---|
| Munich | 4 September 2020 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 3627

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2020

| Patent document cited in search report | Publication date | Patent family member(s) | Publication date |
|---|---|---|---|
| WO 2013011479 A2 | 24-01-2013 | BR 112014000965 A2 | 13-06-2017 |
| | | CN 103649337 A | 19-03-2014 |
| | | CN 109852671 A | 07-06-2019 |
| | | DK 2734643 T3 | 20-03-2017 |
| | | DK 3173489 T3 | 10-12-2018 |
| | | EP 2549399 A1 | 23-01-2013 |
| | | EP 2734643 A2 | 28-05-2014 |
| | | EP 3173489 A1 | 31-05-2017 |
| | | EP 3418396 A1 | 26-12-2018 |
| | | ES 2617195 T3 | 15-06-2017 |
| | | ES 2700617 T3 | 18-02-2019 |
| | | JP 6204354 B2 | 27-09-2017 |
| | | JP 2014520566 A | 25-08-2014 |
| | | JP 2017205129 A | 24-11-2017 |
| | | JP 2019129850 A | 08-08-2019 |
| | | KR 20140046464 A | 18-04-2014 |
| | | MX 352827 B | 11-12-2017 |
| | | RU 2014106026 A | 27-08-2015 |
| | | US 2014156200 A1 | 05-06-2014 |
| | | WO 2013011479 A2 | 24-01-2013 |
| WO 2014102668 A2 | 03-07-2014 | AU 2013368945 A1 | 13-08-2015 |
| | | AU 2020202706 A1 | 14-05-2020 |
| | | BR 112015015131 A2 | 11-07-2017 |
| | | CA 2896414 A1 | 03-07-2014 |
| | | CN 105074005 A | 18-11-2015 |
| | | EP 2938745 A2 | 04-11-2015 |
| | | JP 6449779 B2 | 09-01-2019 |
| | | JP 6721665 B2 | 15-07-2020 |
| | | JP 2016509472 A | 31-03-2016 |
| | | JP 2019076096 A | 23-05-2019 |
| | | KR 20150100896 A | 02-09-2015 |
| | | RU 2015131009 A | 30-01-2017 |
| | | US 2015347672 A1 | 03-12-2015 |
| | | WO 2014102668 A2 | 03-07-2014 |
| US 2009203534 A1 | 13-08-2009 | DE 102005050933 A1 | 26-04-2007 |
| | | EP 2004846 A2 | 24-12-2008 |
| | | US 2009203534 A1 | 13-08-2009 |
| | | WO 2007045197 A2 | 26-04-2007 |
| WO 2019068623 A1 | 11-04-2019 | CA 3077501 A1 | 11-04-2019 |
| | | CN 111587293 A | 25-08-2020 |
| | | EP 3692170 A1 | 12-08-2020 |
| | | US 2020240979 A1 | 30-07-2020 |
| | | WO 2019068623 A1 | 11-04-2019 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 20 16 3627

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2020

| Patent document cited in search report | Publication date | Patent family member(s) | Publication date |
|---|---|---|---|

EPO FORM P0459

For more details about this annex : see Official Journal of the European Patent Office, No. 12/82

## REFERENCES CITED IN THE DESCRIPTION

**Patent documents cited in the description**

- WO 2013011479 A
- WO 2014102668 A
- WO 2015101635 A
- WO 2016062891 A
- WO 2017029215 A
- WO 2014174003 A
- WO 2016062892 A
- WO 2016062893 A
- WO 2018096076 A
- WO 2019068585 A
- WO 2019120658 A
- WO 2019068543 A
- WO 2019068562 A
- WO 2019068623 A
- WO 2013011479 A2
- WO 2014102668 A2

**Non-patent literature cited in the description**

- **W VERHAEGH ; A VAN DE STOLPE.** *Oncotarget,* 2014, vol. 5 (14), 5196
- Prognostic and predictive enrichment in sepsis. **N. L. STANSKI ; H. R. WONG.** Nature Reviews Nephrology. Nature Publishing Group, 01 January 2019
- **N. K. PATIL ; J. K. BOHANNON ; E. R. SHERWOOD.** Immunotherapy: A promising approach to reverse sepsis-induced immunosuppression. *Pharmacol. Res.,* 2016, vol. 111, 688-702
- **R. S. HOTCHKISS ; G. MONNERET ; D. PAYEN.** Sepsis-induced immunosuppression: from cellular dysfunctions to immunotherapy. *Nat. Rev. Immunol.,* December 2013, vol. 13 (12), 862-74
- **M. R. GUBBELS BUPP ; T. N. JORGENSEN.** Androgen-Induced Immunosuppression. *Front. Immunol.,* April 2018, vol. 9, 794
- **A. TRIGUNAITE ; J. DIMO.** Suppressive effects of androgens on the immune system. *Cell. Immunol.,* April 2015, vol. 294 (2), 87-94
- **DIPL BIOCHEM DANIELA RÖLL.** *Androgen-regulation of sepsis response: Beneficial role of androgen receptor antagonists*
- **F. FATTAHI ; P. A. WARD.** Understanding Immunosuppression after Sepsis. *Nat. Rev. Mol. Cell Biol,* 2017, vol. 42, 51-65
- **A. ROQUILLY et al.** Local Modulation of Antigen-Presenting Cell Development after Resolution of Pneumonia Induces Long-Term Susceptibility to Secondary Infections. *Immunity,* 05 July 2017, vol. 47 (1), 135-147
- **M. CECCONI et al.** Sepsis and septic shock. *Lancet,* 2018, vol. 392, 75-87